# EUROPEAN PATENT SPECIFICATION

(11) **EP 0 915 861 B1**
(45) Date of publication and mention of the grant of the patent: **05.12.2001**
(21) Application number: 97932407.6
(22) Date of filing: 30.06.1997
(51) Int. Cl.: C07D 301/10, C07D 301/06, B01J 23/66, B01J 29/89

(54) **PROCESS FOR THE DIRECT OXIDATION OF OLEFINS TO OLEFIN OXIDES**
VERFAHREN ZUR DIREKTEN OXIDATION VON OLEFINEN ZU OLEFINOXIDEN
PROCEDE POUR OXYDER DIRECTEMENT LES OLEFINES EN OXYDES D'OLEFINES

(30) Priority: 01.07.1996 US 21013 P; 11.07.1996 US 679605; 20.09.1996 US 26590 P; 20.09.1996 US 26591 P
(43) Date of publication of application: 19.05.1999
(73) Proprietor: THE DOW CHEMICAL COMPANY, Midland, Michigan 48674 (US)
(72) Inventor: BOWMAN, Robert, G., Midland, MI 48640 (US); MAJ, Joseph, J., Midland, MI 48640 (US); CLARK, Howard, W., Midland, MI48642 (US); HARTWELL, George, E., Midland, MI 48642 (US); WOMACK, Joseph, L., Oakland, CA 94618 (US); BARE, Simon, R., Glen Ellyn, IL 60137 (US)
(74) Representative: Burford, Anthony Frederick
(86) International application number: PCT/US97/11415
(87) International publication number: WO 98/00414

(56) References cited:
- EP-A- 0 709 360
- EP-A- 0 723 810
- DE-A- 4 425 672
- GB-A- 1 327 497
- GB-A- 1 338 901
- US-A- 4 839 327
- US-A- 4 937 219
- US-A- 5 506 273
- DATABASE WPI Section Ch, Week 9512 Derwent Publications Ltd., London, GB; Class E36, AN 95-084550 XP002042443 & JP 07 008 797 A (AGENCY OF IND SCI & TECHNOLOGY) , 13 January 1995
- EP-A-0 827 779

## Description

This invention pertains to a process and catalyst for the direct oxidation of olefins, such as propylene, by oxygen to olefin oxides, such as propylene oxide.

Olefin oxides, such as propylene oxide, are used to alkoxylate alcohols to form polyether polyols, such as polypropylene polyether polyols, which find significant utility in the manufacture of polyurethanes and synthetic elastomers. Olefin oxides are also important intermediates in the manufacture of alkylene glycols, such as propylene glycol and dipropylene glycol, and alkanolamines, such as isopropanolamine, which are useful as solvents and surfactants.

Propylene oxide is produced commercially via the well-known chlorohydrin process wherein propylene is reacted with an aqueous solution of chlorine to produce a mixture of propylene chlorohydrins. The chlorohydrins are dehydrochlorinated with an excess of alkali to produce propylene oxide. This process suffers from the production of a low concentration salt stream. (See K. Weissermel and H. J. Arpe, *Industrial Organic Chemistry*, 2^{nd} ed., VCH Publishers, Inc., New York, NY, 1993, pp. 264-265.)

Another well-known route to olefin oxides relies on the transfer of an oxygen atom from an organic hydroperoxide or peroxycarboxylic acid to an olefin. In the first step of this oxidation route, a peroxide generator, such as isobutane or acetaldehyde, is autoxidized with oxygen to form a peroxy compound, such as t-butyl hydroperoxide or peracetic acid. This compound is used to epoxidize the olefin, typically in the presence of a transition metal catalyst, including titanium, vanadium, molybdenum, and other heavy metal compounds or complexes. Along with the olefin oxide produced, this process disadvantageously produces equimolar amounts of a coproduct, for example an alcohol, such as t-butanol, or an acid, such as acetic acid, whose value must be captured in the market place. (*Industrial Organic Chemistry, ibid.*, pp. 265-269.)

Although the direct oxidation of ethylene by molecular oxygen to ethylene oxide has been commercialized with a silver catalyst, it is known that the analogous direct oxidation of propylene exhibits a low selectivity to the olefin oxide. Disadvantageously large amounts of acrolein and oxygen-containing C₁₋₃ byproducts are produced. (*Industrial Organic Chemistry, ibid.*, p. 264.) Some patents represented by US-A-4,007,135 and US-A-4,845,253, teach the use of metal-promoted silver catalysts for the oxidation of propylene with oxygen to propylene oxide. Among the metal promoters disclosed are gold, beryllium, magnesium, calcium, barium, strontium, and the rare earth lanthanides. These promoted silver catalysts also exhibit low selectivities to the olefin oxide.

Alternatively, EP-A1-0,709,360 discloses a process of oxidizing an unsaturated hydrocarbon, such as propylene, with oxygen in the presence of hydrogen and a catalyst to form an epoxide, such as propylene oxide. Gold deposited on titanium dioxide, preferably the anatase phase of crystalline titanium dioxide, further immobilized on a carrier such as silica or alumina, is taught as the catalyst composition. The catalyst exhibits lower olefin oxide selectivity and less efficient hydrogen consumption when operated at higher temperatures. Additionally, the catalyst has a short run time.

PCT publication WO-A1-96/02323 discloses the oxidation of an olefin, including propylene, with oxygen in the presence of hydrogen and a catalyst to form an olefin oxide. The catalyst is a titanium or vanadium silicalite containing at least one platinum group metal, and optionally, an additional metal selected from gold, iron, cobalt, nickel, rhenium, and silver. The productivity of olefin oxide is low in this process.

In view of the above, a need continues to exist in the chemical industry for an efficient direct route to propylene oxide and higher olefin oxides from the reaction of oxygen with C₃ and higher olefins. The discovery of such a process which simultaneously achieves high selectivity to the olefin oxide at an economically advantageous conversion of the olefin would represent a significant achievement over the prior art. For commercial viability such a process would also require that the catalyst exhibit a long lifetime.

US-A-4,839,327 and US-A-4,937,219 represent additional art disclosing a composition comprising gold particles having a particle size smaller than 500 Å immobilized on an alkaline earth oxide or titanium dioxide or a composite oxide of titanium dioxide with an alkaline earth oxide. A preparation of this composition involves deposition of a gold compound onto the alkaline earth oxide, titanium oxide, or composite oxide followed by calcination to form metallic gold having a particle size smaller than 500 Å. This teaching is silent with respect to a process for producing an olefin oxide.

US-A-5,506273 and JP-A-07008797 disclose gold-titanium catalysts with different types of support prepared using sodium hydroxide to balance the pH. The sodium ions are then extensively washed-out.

This invention is a novel process of preparing an olefin oxide directly from an olefin and oxygen. The process comprises contacting an olefin having at least three carbon atoms with oxygen in the presence of hydrogen and a catalyst under process conditions sufficient to produce the corresponding olefin oxide. The unique catalyst which is employed in the process of this invention comprises gold, at least one promoter metal, and a titanium-containing support.

The novel process of this invention is useful for producing an olefin oxide directly from oxygen and an olefin having three or more carbon atoms. Unexpectedly, the process of this invention produces the olefin oxide in a remarkably high selectivity. Partial and complete combustion products, such as acrolein and carbon dioxide, which are found in large amounts in many prior art processes, are produced in lesser amounts in the process of this invention. Significantly, the process of this invention can be operated at a high temperature, specifically greater than 120°C, while still maintaining high selectivity to the olefin oxide. Operation at higher temperatures advantageously provides steam credits from the heat produced. Accordingly, the process of this invention can be integrated into a total plant design wherein the heat derived from the steam is used to drive additional processes, such as the separation of the olefin oxide from water. Since water is produced as a coproduct in this process, as a further advantage, the hydrogen efficiency in the process of this invention, as measured by the water to olefin oxide molar ratio, is good. Most advantageously, the process in preferred embodiments exhibits an olefin conversion which is good.

In another aspect, this invention is a unique catalyst composition comprising gold, at least one promoter metal, and a titanium-containing support. The promoter metal is selected from Group 1, Group 2, the rare earth lanthanide metals, and the actinide metals, and combinations thereof. The following proviso applies. The composition of this invention does not include gold on a Group 2 promoter metal titanate.

The novel composition of this invention can be effectively used in the aforementioned direct oxidation of an olefin having three or more carbon atoms to the corresponding epoxide. Besides being active and highly selective for the olefin oxide, the catalyst exhibits evidence of a long lifetime. As a further advantage, when partially or completely spent, the catalyst is easy to regenerate. Accordingly, this unique composition possesses highly desirable properties for catalyzing the direct oxidation of propylene and higher olefins to their corresponding olefin oxides.

The novel process of this invention comprises contacting an olefin having three or more carbon atoms with oxygen in the presence of hydrogen and an epoxidation catalyst under process conditions sufficient to prepare the corresponding olefin oxide. In one preferred embodiment, a diluent is employed, as described in detail hereinafter. The relative molar quantities of olefin, oxygen, hydrogen, and optional diluent can be any which are sufficient to prepare the desired olefin oxide. In a preferred embodiment of this invention, the olefin employed is a C₃₋₁₂ olefin, and it is converted to the corresponding C₃₋₁₂ olefin oxide. In a more preferred embodiment, the olefin is a C₃₋₈ olefin, and it is converted to the corresponding C₃₋₈ olefin oxide. In a most preferred embodiment, the olefin is propylene, and the olefin oxide is propylene oxide.

The novel catalyst which is employed in the process of this invention comprises gold, at least one promoter metal, and a titanium-containing support. In a preferred embodiment of the process, the promoter metal is selected from Group 1, Group 2, the rare earth lanthanides, and the actinide metals of the Periodic Table of the Elements, as referenced in the *CRC Handbook of Chemistry and Physics,* 75th edition, CRC Press, 1994-1995. Combinations of the aforementioned metals can also be employed.

Any olefin containing three or more carbon atoms can be employed in the process of this invention. Monoolefins are preferred, but compounds containing two or more olefins, such as dienes, can also be used. The olefin can be a simple hydrocarbon containing only carbon and hydrogen atoms; or alternatively, the olefin can be substituted at any of the carbon atoms with an inert substituent. The term "inert", as used herein, requires the substituent to be substantially non-reactive in the process of this invention. Suitable inert substituents include, but are not limited to, halides, ether, ester, alcohol, and aromatic moieties, preferably chloro, C₁₋₁₂ ether, ester, and alcohol moieties and C₆₋₁₂ aromatic moieties. Non-limiting examples of olefins which are suitable for the process of this invention include propylene, 1-butane, 2-butene, 2-methylpropene, 1-pentene, 2-pentene, 2-methyl-1-butene, 2-methyl-2-butene, 1-hexene, 2-hexene, 3-hexene, and analogously, the various isomers of methylpentene, ethylbutene, heptene, methylhexene, ethylpentene, propylbutene, the octenes, including preferably 1-octene, and other higher analogues of these; as well as butadiene, cyclopentadiene, dicyclopentadiene, styrene, α-methylstyrene, divinylbenzene, allyl chloride, allyl alcohol, allyl ether, allyl ethyl ether, allyl butyrate, allyl acetate, allyl benzene, allyl phenyl ether, allyl propyl ether, and allyl anisole. Preferably, the olefin is an unsubstituted or substituted C₃₋₁₂ olefin, more preferably, an unsubstituted or substituted C₃₋₈ olefin. Most preferably, the olefin is propylene. Many of the aforementioned olefins are available commercially; others can be prepared by chemical processes known to those skilled in the art.

The quantity of olefin employed in the process can vary over a wide range provided that the corresponding olefin oxide is produced. Generally, the quantity of olefin depends upon the specific process features, including for example, the design of the reactor, the specific olefin, and economic and safety considerations. Those skilled in the art will know how to determine a suitable range of olefin concentrations for the specific process features. Typically, on a molar basis an excess of olefin is used relative to the oxygen, because this condition enhances the productivity to olefin oxide. In light of the disclosure herein, the quantity of olefin is typically greater than 1, preferably, greater than 10, and more preferably, greater than 20 mole percent, based on the total moles of olefin, oxygen, hydrogen, and optional diluent. Typically, the quantity of olefin is less than 99, preferably, less than 85, and more preferably, less than 70 mole percent, based on the total moles of olefin, oxygen, hydrogen, and optional diluent.

Oxygen is also required for the process of this invention. Any source of oxygen is acceptable, including air and essentially pure molecular oxygen. Other sources of oxygen may be suitable, including ozone and nitrogen oxides, such as nitrous oxide. Molecular oxygen is preferred. The quantity of oxygen employed can vary over a wide range provided that the quantity is sufficient for producing the desired olefin oxide. Ordinarily, the number of moles of oxygen per mole of olefin is less than 1. Under these conditions the selectivity to olefin oxide is enhanced while the selectivity to combustion products, such as carbon dioxide, is minimized. Preferably, the quantity of oxygen is greater than 0.01, more preferably, greater than 1, and most preferably greater than 5 mole percent, based on the total moles of olefin, hydrogen, oxygen, and optional diluent. Preferably, the quantity of oxygen is less than 30, more preferably, less than 25, and most preferably less than 20 mole percent, based on the total moles of olefin, hydrogen, oxygen, and optional diluent. Above 20 mole percent, the concentration of oxygen may fall within the flammable range for olefin-hydrogen-oxygen mixtures.

Hydrogen is also required for the process of this invention. In the absence of hydrogen, the activity of the catalyst is significantly decreased. Any source of hydrogen can be used in the process of this invention including for example, molecular hydrogen obtained from the dehydrogenation of alkanes and alcohols. In an alternative embodiment, the hydrogen may be generated *in situ* in the olefin oxidation process, for example, by dehydrogenating alkanes, such as propane or isobutane, or alcohols, such as isobutanol. Alternatively, hydrogen can be used to generate a catalyst-hydride complex or a catalyst-hydrogen complex which can supply the necessary hydrogen to the process.

Any quantity of hydrogen can be employed in the process provided that the amount is sufficient to produce the olefin oxide. Suitable quantities of hydrogen are typically greater than 0.01, preferably, greater than 0.1, and more preferably, greater than 3 mole percent, based on the total moles of olefin, hydrogen, oxygen, and optional diluent. Suitable quantities of hydrogen are typically less than 50, preferably, less than 30, and more preferably, less than 20 mole percent, based on the total moles of olefin, hydrogen, oxygen, and optional diluent.

In addition to the above reagents, it may be desirable to employ a diluent in the reaction mixture, although the use thereof is optional. Since the process of this invention is exothermic, a diluent beneficially provides a means of removing and dissipating the heat produced. In addition, the diluent provides an expanded concentration regime in which the reactants are non-flammable. The diluent can be any gas or liquid which does not inhibit the process of this invention. The specific diluent chosen will depend upon the manner in which the process is conducted. For example, if the process is conducted in a gas phase, then suitable gaseous diluents include, but are not limited to, helium, nitrogen, argon, methane, carbon dioxide, steam, and mixtures thereof. Most of these gases are essentially inert with respect to the process of this invention. Carbon dioxide and steam may not necessarily be inert, but may have a beneficial promoting effect. If the process is conducted in a liquid phase, then the diluent can be any oxidation stable and thermally stable liquid. Suitable liquid diluents include chlorinated aromatics, preferably chlorinated benzenes, such as chlorobenzene and dichlorobenzene; C₁₋₁₀ aliphatic alcohols; chlorinated aliphatic alcohols, preferably C₁₋₁₀ chlorinated alkanols, such as chloropropanol; as well as liquid polyethers, polyesters, and polyalcohols.

If used, the amount of diluent is typically greater than 0, preferably greater than 0.1, and more preferably, greater than 15 mole percent, based on the total moles of olefin, oxygen, hydrogen, and optional diluent. The amount of diluent is typically less than 90, preferably, less than 80, and more preferably, less than 70 mole percent, based on the total moles of olefin, oxygen, hydrogen, and diluent.

The concentrations of olefin, oxygen, hydrogen, and diluent disclosed hereinabove are suitably based on the reactor designs and process parameters disclosed herein. Those skilled in the art will recognize that concentrations other than those disclosed herein may be suitably employed in other various engineering realizations of the process.

The unique catalyst which is beneficially employed in the process of this invention comprises gold, at least one promoter metal, and a titanium-containing support. The gold can exist in discrete gold particles and/or in mixed gold-promoter metal particles. The gold can exist in the zerovalent state or in a positive valence state ranging from greater than 0 to +3, as determined by X-ray absorption spectroscopy or X-ray photoelectron spectroscopy. Typically, the average size (or diameter) of the gold particles or the mixed gold-promoter metal particles is at least 10 Å, as measured by transmission electron microscopy (TEM). Smaller sized gold and/or gold-promoter metal entities may be found dispersed over the surface of the support. Preferably, the average gold particle size is greater than 10 Å, more preferably, greater than 12 Å, and most preferably, greater than 25 Å. Preferably, the average gold particle size is less than 500 Å, more preferably, less than 200 Å, and most preferably, less than 100 Å.

The titanium-containing support may take a variety of forms. The titanium predominantly exists in a positive oxidation state, as determined by X-ray photoelectron and X-ray absorption spectroscopies. More preferably, the titanium exists in an oxidation state of +2 or higher, most preferably, in an oxidation state of +3 to +4. Non-limiting examples of titanium-containing supports which can be suitably employed in the catalyst of this invention include those described hereinbelow. Titanium-containing supports noted hereinbelow which do not contain the desired promoter metal(s) must be treated to incorporate the promoter metal(s) into or onto the support. Supports already containing promoter metals may or may not require extra promoter metal(s) to be added to the support.

### a. Titanium Dioxide

Amorphous and crystalline titanium dioxide can be suitably employed as the titanium-containing support. The crystalline phases include anatase, rutile, and brookite. Included in this category are composites comprising titanium dioxide supported on silica.

### b. Promoter Metal Titanates

Stoichiometric and non-stoichiometric compounds comprising promoter metal titanates can also be suitably employed as the catalyst support The promoter metal titanates can be crystalline or amorphous. Non-limiting examples of these include the titanates of Group 1, Group 2, and the lanthanide and actinide metals. Preferably, the promoter metal titanate is selected from magnesium titanate, calcium titanate, barium titanates, strontium titanate, sodium titanate, potassium titanate, and the titanates of erbium, lutetium, thorium, and uranium.

### c. Titanosilicates

Crystalline and amorphous titanosilicates, preferably those that are porous, are also suitably employed as the support. Titanosilicates possess a framework structure formed from SiO₄⁴⁻ wherein a portion of the silicon atoms is replaced by titanium. Within the framework structure of porous titanosilicates there exists a regular or irregular system of pores and/or channels. Empty cavities, referred to as cages, can also be present. The pores can be isolated or interconnecting, and they can be one, two, or three dimensional. The pores are more preferably micropores or mesopores or some combination thereof. As used herein, a micropore has a pore diameter (or critical dimension as in the case of a non-circular perpendicular cross-section) ranging from 4 Å to 20 Å, while a mesopore has a pore diameter or critical dimension ranging from greater than 20 Å to 200 Å. The combined volume of the micropores and the mesopores preferably comprises 70 percent or greater of the total pore volume, and more preferably, 80 percent or greater of the total pore volume. The balance of the pore volume will comprise macropores, which have a pore diameter of greater than 200 Å. Macropores include the void spaces between particles or crystallites.

The pore diameter (or critical dimension), pore size distribution, and surface area of the porous titanosilicate can be obtained from the measurement of adsorption isotherms and pore volume. Typically, the measurements are made on the titanosilicate in powder form using as an adsorbate nitrogen at 77 K or argon at 88 K and using any suitable adsorption analyzer, such as a Micromeritics ASAP 2000 instrument. Measurement of micropore volume is derived from the adsorption volume of pores having a diameter in the range from 4 Å to 20 Å. Likewise, measurement of mesopore volume is derived from the adsorption volume of pores having a diameter in the range from greater than 20 Å to 200 Å. From the shape of the adsorption isotherm, a qualitative identification of the type of porosity, for example, microporous or macroporous, can be made. Additionally, increased porosity can be correlated with increased surface area. Pore diameter (or critical dimension) can be calculated from the data using equations described by Charles N. Satterfield in *Heterogeneous Catalysis in Practice,* McGraw-Hill Book Company, New York, 1980, pp. 106-114.

Additionally, crystalline porous titanosilicates can be identified by X-ray diffraction methods (XRD), either by comparing the XRD pattern of the material of interest with a previously published standard or by analyzing the XRD pattern of a single crystal to determine framework structure, and if pores are present, the pore geometry and pore size.

Non-limiting examples of porous titanosilicates which are suitably employed in the process of this invention include porous amorphous titanosilicates; porous layered titanosilicates; crystalline microporous titanosilicates, such as titanium silicalite-1 (TS-1), titanium silicalite-2 (TS-2), titanosilicate beta (Ti-beta), titanosilicate ZSM-12 (Ti-ZSM-12) and titanosilicate ZSM-48 (Ti-ZSM-48); as well as mesoporous titanosilicates, such as Ti-MCM-41.

The pore structure of TS-1 comprises two interconnecting, roughly cylindrical, 10-ring pores of about 5 Å diameter. A 10-ring pore is formed from a total of ten tetrahedra (SiO₄⁴⁻ and TiO₄⁴⁻). Titanium silicalite and its characteristic XRD pattern have been reported in US-A-4,410,501. TS-1 can be obtained commercially, but it can also be synthesized following the methods described in US-A-4,410,501. Other preparations have been reported by the following: A. Tuel, *Zeolites,* 1996, **16**, 108-117; by S. Gontier and A. Tuel, *Zeolites,* 1996, **16**, 184-195; by A. Tuel and Y. Ben Taarit in *Zeolites,* 1993, **13**, 357-364; by A. Tuel, Y. Ben Taarit and C. Naccache in *Zeolites*, 1993, **13**, 454-461; by A. Tuel and Y. Ben Taarit in *Zeolites,* 1994, **14**, 272-281; and by A. Tuel and Y. Ben Taarit in *Microporous Materials,* 1993, **1**, 179-189.

The pore structure of TS-2 comprises one three-dimensional, 10-ring microporous system. TS-2 can be synthesized by the methods described in the following references: J. Sudhakar Reddy and R. Kumar, *Zeolites,* 1992, **12**, 95-100; by J. Sudhakar Reddy and R. Kumar, *Journal of Catalysis,* 1991, **130**, 440-446; and by A. Tuel and Y. Ben Taarit, *Applied Catal. A,* General, 1993, **102**, 69-77.

The pore structure of Ti-beta comprises two interconnecting 12-ring, roughly cylindrical pores of about 7 A diameter. The structure and preparation of titanosilicate beta have been described in the following references: PCT patent publication WO-A-94/02245 (1994); M. A. Camblor, A. Corma, and J.H. Perez-Pariente, *Zeolites,* 1993, **13**, 82-87; and M. S. Rigutto, R. de Ruiter, J. P. M. Niederer, and H. van Bekkum, *Stud. Surf. Sci. Cat.,* 1994, **84**, 2245-2251.

The pore structure of Ti-ZSM-12 comprises one, one-dimensional 12-ring channel system of dimensions 5.6 x 7.7 Å, as referenced by S. Gontier and A. Tuel, *ibid.*

The pore structure of Ti-ZSM-48 comprises a one-dimensional 10-ring channel system of dimensions 5.3 Å by 5.6 Å, as referenced by R. Szostak, *Handbook of Molecular Sieves,* Chapman & Hall, New York, 1992, pp. 551-553. Other references to the preparation and properties of Ti-ZSM-48 include C. B. Dartt, C. B. Khouw, H. X. Li, and M. E. Davis, *Microporous Materials*, 1994, **2**, 425-437; and A. Tuel and Y. Ben Taarit, *Zeolites,* 1996, **15**, 164-170.

Ti-MCM-41 is a hexagonal phase isomorphous to the aluminosilicate MCM-41. The channels in MCM-41 are one-dimensional with diameters ranging from 28 Å to 100 Å. Ti-MCM-41 can be prepared as described in the following citations: S. Gontier and A. Tuel, *Zeolites,* 1996, **15**, 601-610; and M. D. Alba, Z. Luan, and J. Klinowski, *J. Phys. Chem.,* 1996, **100**, 2178-2182.

The silicon to titanium atomic ratio (Si/Ti) of the titanosilicate can be any ratio which provides for an active and selective epoxidation catalyst in the process of this invention. A generally advantageous Si/Ti atomic ratio is equal to or greater than 5/1, preferably, equal to or greater than 10/1. A generally advantageous Si/Ti atomic ratio is equal to or less than 200/1, preferably, equal to or less than 100/1. It is noted that the Si/Ti atomic ratio defined herein refers to a bulk ratio which includes the total of the framework titanium and the extra-framework titanium. At high Si/Ti ratios, for example, about 100/1 or more, there may be little extra-framework titanium and the bulk ratio essentially corresponds to the framework ratio.

In one preferred embodiment of this invention, the titanosilicate is substantially free of crystalline titanium dioxide, present for example as extra-framework titania or as an added support or carrier. Raman spectroscopy can be used to detect the presence of crystalline titanium dioxide. The anatase phase of titanium dioxide exhibits a characteristic strong, sharp Raman peak at about 147 cm⁻¹. The rutile phase of titanium dioxide exhibits peaks at about 448 cm⁻¹ and about 612 cm⁻¹. The brookite phase, which usually is available only as a natural mineral, exhibits a characteristic peak at about 155 cm⁻¹. The rutile and brookite peaks have a lower intensity than the 147 cm⁻¹ peak of anatase. In the preferred embodiment noted herein, Raman peaks for the anatase, rutile, and brookite phases of titanium dioxide are essentially absent. When the catalyst exhibits essentially no detectable peaks at the aforementioned wavenumbers, then it is estimated that less than 0.02 weight percent of the catalyst exists in the form of crystalline titanium dioxide. Raman spectra can be obtained on any suitable laser Raman spectrometer equipped, for example, with an argon ion laser having an excitation line at 514.5 nm, 532 nm, and/or 785 nm and having a laser power of 90 to 100 mW measured at the sample.

### d. Titanium Dispersed on Silica

Another suitable support for the catalyst of this invention comprises titanium dispersed on silica. This support can be obtained commercially, or alternatively, prepared by the methods described hereinbelow. A preferred form of this support is described in European Patent Application Number 97933228.2, the corresponding European Regional phase application of PCT International Application Number PCT/US97/11417, published 8 January 1998 as Publication Number WO 98/00415.

In the aforementioned preferred support, the titanium ions are dispersed over the surface of the silica substantially in a disorganized phase. The term "substantially" means that greater than 80 weight percent of the titanium exists in the disorganized phase. Preferably, greater than 85, even more preferably, greater than 90, and most preferably, greater than 95 weight percent of the titanium exists in the disorganized phase. This result implies that typically less than 20, preferably, less than 15, even more preferably, less than 10, and most preferably, less than 5 weight percent of the titanium in the support exists in an organized crystalline form, specifically crystalline titanium dioxide. Thus, in its typical form, the support is substantially free of crystalline titanium dioxide, and in its most preferred form, essentially free of crystalline titanium dioxide. In another preferred embodiment, the gold particles are preferentially associated with the disorganized titanium phase rather than with any crystalline phase of titanium dioxide which may be present. HR-TEM and Energy Dispersive X-ray analysis (EDX) can be used to image the association of gold particles with titanium.

The titanium ions in the disorganized phase may be isolated from other titanium ions, or alternatively, the titanium ions may be linked through oxide bonds to other titanium ions in small domains of a two-dimensional monolayer network. Whatever its actual topology, the disorganized phase does not exhibit an organized, periodic crystallinity. In another aspect of this invention, the titanium ions preferably occupy sites of substantially four or five-fold coordination or distorted variations thereof, as opposed to octahedral coordination. In its broadest concept, however, the disorganized phase of titanium is not limited to any particular topology or coordination.

The disorganized titanium phase can be distinguished from bulk crystalline titanium dioxide by high resolution transmission electron microscopy (HR-TEM) and/or by Raman spectroscopy, as described hereinbelow. Additionally, the disorganized phase does not exhibit a distinct X-ray diffraction (XRD) pattern. X-ray diffraction (XRD), however, is less sensitive in detecting crystalline titanium dioxide. Accordingly, the absence of an XRD pattern characteristic of the bulk crystalline phases of titanium dioxide is not conclusive evidence that these phases are absent in the support. Ultraviolet-Visible Diffuse Reflectance Spectroscopy (UV-VIS DRS) provides a third analytical technique which can distinguish between the disorganized titanium phase and crystalline titanium dioxide. Typically, any one of HR-TEM, Raman, or UV-VIS DRS can be used to identify the disorganized phase. Preferably, two or more of these methods are used to identify the disorganized phase. Additionally, titanium K-edge X-Ray Absorption Near Edge Structure (XANES) spectroscopy can be used in a complementary manner with HR-TEM, Raman and/or UV-VIS DRS to identify the disorganized phase. It is further noted that titanium L₂-edge and L₃-edge XANES and oxygen K-edge XANES spectroscopies can provide additional data which are consistent with the aforementioned techniques and with differences between the disorganized phase and crystalline titanium dioxide.

Any high resolution transmission electron microscope can be used to image the catalyst or support of this invention. The term "high resolution" implies resolution at the level of atomic lattices. Accordingly, the point to point resolution of the instrument should be at least 2 Å or better. The preferred catalyst or support of this type exhibits essentially no discemible regular pattern, an image consistent with a disorganized phase. In contrast, a catalyst or support containing crystalline titanium dioxide exhibits images of lattice planes separated by about 3.5 Å for anatase and about 3.25 Å for rutile.

Raman spectroscopy is also sensitive to the presence of crystalline titanium dioxide, as noted in section (c) hereinabove. In the support disclosed herein, the Raman peaks for the anatase, rutile, and brookite phases of titanium dioxide are essentially absent.

The UV-VIS DRS spectrum of the support of this type can be obtained on any instrument designed for that purpose, for example, a DRS spectrometer Model UV-3101PC scanning from 200 to 800 nm. The spectrum comprises a convolution of bands due to oxygen to titanium charge transfer in the 300 nm region, Mie scattering of gold particles in the 525 nm region, and other bands attributed to scattering by gold particles or absorption by organic species found on used catalyst samples. Deconvolution of the spectra into its separate components can be accomplished by non-linear least squares fitting. The charge transfer region is particularly useful, and its analysis has been previously described by S. Klein et al., in the *Journal of Catalysis*, **163**, 489-491 (1996). The fresh catalyst or support containing disorganized titanium exhibits the charge transfer band at 310 nm or lower wavelengths. In contrast, a catalyst or support containing crystalline titanium dioxide exhibits the charge transfer band at 315 nm or higher wavelengths. For example, the pure anatase and rutile phases of titanium dioxide exhibit a peak at 359 nm and 395 nm, respectively.

Titanium K-edge XANES is also useful in distinguishing between the disorganized titanium phase and the anatase and rutile phases of titanium dioxide. Measurement of the XANES spectrum is described hereinbelow. Both anatase and rutile titanium exhibit three peaks in the Ti K-edge XANES. When the instrument is run in transmission mode and calibrated with an internal metallic titanium standard wherein zero energy is set at 4,966.0 eV, anatase and rutile each exhibit three peaks at about +2.9, +5.9, and +8.3 eV. In anatase and rutile the titanium coordination is distorted octahedral. In contrast, the disorganized titanium phase of this invention exhibits substantially a single peak at about +4.6 ± 1.2 eV, preferably, +4.6 ± 0.3 eV. The titanium coordination in the disorganized phase appears to be closer to four or five-fold coordination.

Any silica can be used in the support provided that it allows for an active catalyst composition. The silicas can be amorphous or crystalline. Preferred silicas are surface hydroxylated. Non-limiting examples of suitable silicas include fumed silica, silica gel, precipitated silicas, precipitated silica gels, silicalite, and mixtures thereof. Preferably, the surface area of the silica is greater than 15 m²/g, more preferably, greater than 20 m²/g, and most preferably, greater than 25 m²/g. More preferably, the surface area of the silica is less than 800 m²/g, most preferably, less than 600 m²/g.

The titanium loading on the silica can be any which gives rise to an active catalyst in the process of this invention. Typically, the titanium loading is greater than 0.02 weight percent, preferably, greater than 0.1 weight percent, based on the weight of the silica. Typically, the titanium loading is less than 20 weight percent, and preferably less than 10 weight percent, based on the weight of the silica.

The method of depositing the titanium ions on the silica is important in obtaining the disorganized titanium phase described hereinabove. A description along the lines of the preparation used herein is given by S. Srinivasan et al. in the *Journal of Catalysis,* **131**, 260-275 (1991), and by R. Castillo et al., *Journal of Catalysis, **161**,524-529* (1996). Generally, the silica support is impregnated with a titanium compound which is reactive with the surface hydroxyls on the silica. Typically, a solution containing a reactive titanium compound is contacted with the silica under mild conditions, such as a temperature between 0°C and 50°C, at about ambient pressure for a time ranging from 30 minutes to 24 hours. Non-limiting examples of suitably reactive titanium compounds include titanium alkoxides, such as titanium isopropoxide, titanium propoxide, titanium ethoxide, and titanium butoxide; titanium sulfate, titanium oxysulfate, titanium halides, preferably titanium chloride; titanium carboxylates, preferably titanium oxalate; and organotitanium halides, such as dicyclopentadiene titanium dichloride, and other organotitanocene dichlorides. Preferably, titanium alkoxides are employed. The solvent can be any which solubilizes the reactive titanium compound, for example, aliphatic alcohols, aliphatic and aromatic hydrocarbons, and water where appropriate. After contacting the support with the solution containing the reactive titanium compound, the support is dried at a temperature between 0°C and 150°C, preferably between 50°C and 150°C, in a vacuum or in a stream of air or an inert gas, such as nitrogen, argon, or helium. Thereafter, the support can be used without calcination or further treatment. Alternatively after drying, the support can be calcined in air or an inert gas, such as nitrogen or helium, to a temperature between 100°C and 800°C, preferably between 100°C and 650°C.

An alternate method of deposition of the titanium is from the gas phase. Volatile titanium compounds, such as titanium chloride, titanium propoxide, or titanium isopropoxide, can be carried through the silica support in a flow of an inert gas such as nitrogen, argon, or helium. The titanium compound can be heated to volatilize or vaporize it into the inert gas stream. The silica support can be heated during the process. Thereafter, the support can be used without calcination or further treatment. Alternatively, the support can be calcined in air or an inert gas, such as nitrogen or helium, to a temperature between 100°C and 800°C, preferably between 100°C and 650°C.

### e. Titanium Dispersed on Promoter Metal Silicates

Yet another suitable support for the catalyst of this invention comprises titanium dispersed on promoter metal silicates. Stoichiometric and non-stoichiometric compounds comprising promoter metal silicates can be used. Any amorphous or crystalline promoter metal silicate is suitably employed. Preferred promoter metal silicates include the silicates of Group 1, Group 2, the lanthanide rare earths, and the actinide metals, and combinations thereof. Non-limiting examples of preferred promoter metal silicates include magnesium silicate, calcium silicate, barium silicate, erbium silicate, and lutetium silicate. The titanium can be dispersed on the promoter metal silicate in a manner analogous to that described in section (d) hereinabove. Analytical methods such as those described in section (d) hereinabove can be used to identify the dispersed titanium phase.

### f. Mixtures of Supports

Any combination or mixture of the supports a-e, described hereinabove, can be employed in the catalyst of this invention.

The gold loading on the titanium-containing supports (a-f) can be any which gives rise to the catalyst of this invention. Generally, the gold loading is greater than 0.01 weight percent, based on the total weight of the gold and support. Preferably, the gold loading is greater than 0.03, more preferably, greater than 0.05 weight percent. Generally, the gold loading is less than 20 weight percent. Preferably, the gold loading is less than 10.0, more preferably, less than 5.0 weight percent.

The gold component can be deposited or supported on the support by any method known in the art which provides for an active and selective epoxidation catalyst in the process of this invention. Non-limiting examples of known deposition methods include impregnation, ion-exchange, and deposition by precipitation. A preferred deposition method is disclosed by S. Tsubota, M. Haruta, T. Kobayashi, A. Ueda, and Y. Nakahara, "Preparation of Highly Dispersed Gold on Titanium and Magnesium Oxide," in *Preparation of Catalysts V*, G. Poncelet, P. A. Jacobs, P. Grange, and B. Delmon, eds., Elsevier Science Publishers B.V., Amsterdam, 1991, p. 695ff. This method involves contacting the support with an aqueous solution of a soluble gold compound at a temperature and pH sufficient to precipitate the gold compound onto the support. Non-aqueous solutions can also be employed. Thereafter, in the preferred method of this invention which is different from the aforementioned reference, the gold/support composite is not washed or is lightly washed, with preferably no more than 100 ml wash liquid per gram composite. Then, the composite is calcined or reduced at a temperature sufficient to reduce the gold substantially to metallic gold having an average particle size between 10 Å and 500 Å.

For aqueous solutions, any water soluble gold compound can be used, such as chloroauric acid, sodium chloroaurate, potassium chloroaurate, gold cyanide, potassium gold cyanide, and diethylamine auric acid trichloride. Typically, the molarity of the soluble gold compound ranges from 0.001 M to the saturation point of the soluble gold compound, preferably, from 0.005 M to 0.5 M. The pH of the aqueous gold solution is adjusted to between 5 and 11, preferably, between 6 and 9, with any suitable base, such as Group 1 metal hydroxides or carbonates, preferably sodium hydroxide, sodium carbonate, potassium carbonate, cesium hydroxide, and cesium carbonate. The desired quantity of support is added to the solution, or vice versa; and if necessary, the pH is again adjusted. Thereafter, the mixture is stirred under air at a temperature between 20°C and 80°C for a time ranging from 1 hour to 24 hours. At the end of this period, the solids are recovered, optionally washed with water, the water optionally containing one or more promoter metal salts preferably at a pH between 5 and 11. Thereafter, the solids are dried under air at a temperature between 80°C and 120°C. The solid is then calcined under air, or calcined in a reducing atmosphere, such as hydrogen, or heated in an inert atmosphere, such as nitrogen, at a temperature between 250°C and 800°C for a time from 1 to 24 hours.

The catalyst which is used in the process of this invention requires at least one promoter metal. Any metal ion having a valence between +1 and +7 which enhances the productivity of the catalyst in the oxidation process of this invention can be employed as a promoter metal. Factors contributing to increased productivity of the catalyst include increased conversion of the olefin, increased selectivity to the olefin oxide, decreased production of water, and increased catalyst lifetime. Non-limiting examples of suitable promoter metal include the metals of Groups 1 through 12 of the Periodic Table of the Elements, as well as the rare earth lanthanides and actinides, as referenced in the *CRC Handbook of Chemistry and Physics,* 75^{th} ed., CRC Press, 1994. Preferably, the promoter metal is selected from Group 1 metals of the Periodic Table including lithium, sodium, potassium, rubidium, and cesium; from Group 2 metals, including beryllium, magnesium, calcium, strontium, and barium; from the lanthanide rare earth metals, including cerium, praseodymium, neodymium, promethium, samarium, europium, gadolinium, terbium, dysprosium, holmium, erbium, thulium, ytterbium, and lutetium; and the actinide metals, specifically, thorium and uranium. More preferably, the promoter metal is magnesium, calcium, barium, erbium, lutetium, lithium, potassium, rubidium, cesium, or a combination thereof.

In another preferred embodiment, the promoter metal excludes palladium, and even more preferably, the promoter metal excludes a Group VIII metal, namely iron, cobalt, nickel, ruthenium, rhodium, palladium, osmium, iridium, and platinum. As used herein, the word "excludes" means that the total concentration of the Group VIII metal(s) is less than 0.01 weight percent, preferably, less than 0.005 weight percent of the total catalyst composition.

The total quantity of promoter metal(s) deposited on the support typically is greater than 0.01, preferably, greater than 0.10, and more preferably, greater than 0.15 weight percent, based on the total weight of the catalyst. The total quantity of promoter metal(s) deposited on the support is generally less than 20, preferably, less than 15, and more preferably, less than 10 weight percent, based on the total weight of the catalyst. Those skilled in the art will recognize that when a promoter metal titanate or silicate is employed, the weight percentage of promoter metal may be much higher, for example, as high as 80 weight percent.

If necessary, the promoter metal(s) can be deposited onto the titanium-containing support simultaneously with the gold, or alternatively, in a separate deposition step either before or after gold is deposited. Alternatively, the promoter metal can be deposited onto a precursor form of the catalyst before the titanium is added, or after it is added, or simultaneously with the titanium. Typically, the promoter metal is deposited from an aqueous or organic solution containing a soluble promoter metal salt. Any salt of the promoter metal with adequate solubility can be used; for example, the metal nitrates, carboxylates, and halides, preferably, the nitrates, are suitable. If an organic solvent is employed, it can be any of a variety of known organic solvents, including, for example, alcohols, esters, ketones, and aliphatic and aromatic hydrocarbons. Ordinarily, the support is contacted with the solution of the promoter metal salt under conditions which are similar to those used for contacting the support with the gold solution. After the promoter metal is deposited, washing is optional, and if done to excess, can leach at least a portion of the promoter metal out of the catalyst Afterwards, calcination under air or under a reducing atmosphere or heating in an inert gas is conducted in a manner similar to that described hereinabove for the gold deposition.

Optionally, the catalyst of this invention can be extruded with, bound to, or supported on a second support, such as silica, alumina, an aluminosilicate, magnesia, titania, carbon, or mixtures thereof. The second support may function to improve the physical properties of the catalyst, such as, the strength or attrition resistance, or to bind the catalyst particles together. Generally, the quantity of second support ranges from 0 to 95 weight percent, based on the combined weight of the catalyst and second support. It is noted that although the catalyst of this invention can be physically mixed or extruded with titania or bound to titania as a second support, in a preferred embodiment the catalyst is substantially free of the anatase phase of titanium dioxide, more preferably free of crystalline titanium dioxide, as noted hereinabove. If titania is used as a second support, however, note that its presence may interfere with the analytical identification of the catalyst. In this instance especially, analysis of the catalyst should be made in the absence of the second support.

The process of this invention can be conducted in a reactor of any conventional design suitable for gas or liquid phase processes. These designs broadly include batch, fixed-bed, transport bed, fluidized bed, moving bed, shell and tube, and trickle bed reactors, as well as continuous and intermittent flow and swing reactor designs. Preferably, the process is conducted in the gas phase and the reactor is designed with heat transfer features for the removal of the heat produced. Preferred reactors designed for these purposes include fluidized bed and moving bed reactors, as well as swing reactors constructed from a plurality of catalyst beds connected in parallel and used in an alternating fashion.

The process conditions for the direct oxidation described herein can vary considerably over a nonflammable and flammable regime. It is beneficial, however, to recognize the conditions which distinguish between nonflammable and flammable mixtures of the olefin, hydrogen, and oxygen. Accordingly, a phase diagram can be constructed or consulted which for any given process temperature and pressure shows the flammable and non-flammable range of reactant compositions, including the diluent, if used. The more preferred reactant mixtures specified hereinabove are believed to lie outside the flammable regime when the process is operated at the more preferred temperatures and pressures specified hereinbelow. Nevertheless, operation within the flammable regime is possible, as designed by one skilled in the art

Usually, the process is conducted at a temperature which is greater than ambient, taken as 20°C, preferably, greater than 70°C, more preferably, greater than 120°C. Usually, the process is conducted at a temperature less than 250°C, preferably less than 225°C, more preferably, less than 200°C. Preferably, the pressure ranges from atmospheric to 400 psig (2758 kPa), more preferably, from 150 psig (1034 kPa) to 250 psig (1724 kPa).

In flow reactors the residence time of the reactants and the molar ratio of reactants to catalyst will be determined by the space velocity. For a gas phase process the gas hourly space velocity (GHSV) of the olefin can vary over a wide range, but typically is greater than 10 ml olefin per ml catalyst per hour (h⁻¹), preferably greater than 100 h⁻¹, and more preferably, greater than 1,000 h⁻¹. Typically, the GHSV of the olefin is less than 50,000 h⁻¹, preferably, less than 35,000 h⁻¹, and more preferably, less than 20,000 h⁻¹. Likewise, for a liquid phase process the weight hourly space velocity (WHSV) of the olefin component may vary over a wide range, but typically is greater than 0.01 g olefin per g catalyst per hour (h⁻¹), preferably, greater than 0.05 h⁻¹, and more preferably, greater than 0.1 h⁻¹. Typically, the WHSV of the olefin is less than 100 h⁻¹, preferably, less than 50 h⁻¹, and more preferably, less than 20 h⁻¹. The gas and weight hourly space velocities of the oxygen, hydrogen, and diluent components can be determined from the space velocity of the olefin taking into account the relative molar ratios desired.

When an olefin having at least three carbon atoms is contacted with oxygen in the presence of hydrogen and the catalyst described herein-above, the corresponding olefin oxide (epoxide) is produced in good productivity. The most preferred olefin oxide produced is propylene oxide.

The conversion of olefin in the process of this invention can vary depending upon the specific process conditions employed, including the specific olefin, temperature, pressure, mole ratios, and form of the catalyst. As used herein the term "conversion" is defined as the mole percentage of olefin which reacts to form products. Generally, the conversion increases with increasing temperature and pressure and decreases with increasing space velocity. Typically, the olefin conversion is greater than 0.1 mole percent, and preferably, greater than 0.2 mole percent, and more preferably, greater than 1.5 percent.

Likewise, the selectivity to olefin oxide can vary depending upon the specific process conditions employed. As used herein, the term "selectivity" is defined as the mole percentage of reacted olefin which forms a particular product, desirably the olefin oxide. Generally, the selectivity to olefin oxide will decrease with increasing temperature and will increase with increasing space velocity. The process of this invention produces olefin oxides in unexpectedly high selectivity. A typical selectivity to olefin oxide in this process is greater than 80 mole percent, preferably, greater than 90 mole percent, and more preferably, greater than 94 mole percent. Even at 165°C the selectivity to propylene oxide is surprisingly high, between 85 and 95 mole percent.

Advantageously, the hydrogen efficiency in the process of this invention is satisfactory. Some additional hydrogen may be burned directly to form water. Accordingly, it is desirable to achieve a water/olefin oxide molar ratio as low as possible. In the process of this invention, the water/olefin oxide molar ratio is typically greater than 2/**1**, but less than 15/1, and preferably, less than 10/**1**, and more preferably, less than 7/**1**.

The catalyst of this invention exhibits evidence of a long lifetime. The term "lifetime" as used herein refers to the time measured from the start of the oxidation process to the point at which the catalyst after regeneration has lost sufficient activity so as to render the catalyst useless, particularly commercially useless. As evidence of its long lifetime, the catalyst remains active for long periods of time with little deactivation. Typically, a run time greater than 125 hours without catalyst deactivation has been achieved in a fixed bed reactor. The preferred run time between regenerations will depend upon the reactor design and may range from minutes for transport bed reactors to several months for fixed bed reactors. As further evidence of its longevity, the catalyst of this invention can be regenerated through multiple cycles without substantial loss in catalyst activity or selectivity.

When its activity has decreased to an unacceptably low level, the catalyst of this invention can be easily regenerated. Any catalyst regeneration method known to those skilled in the art can be applied to the catalyst of this invention, provided that the catalyst is reactivated for the oxidation process described herein. One regeneration method comprises heating the deactivated catalyst at a temperature between 150°C and 500°C in a regeneration gas containing hydrogen and/or oxygen and optionally an inert gas. A preferred regeneration temperature lies between 200°C and 400°C. The amounts of hydrogen and/or oxygen in the regeneration gas can be any which effectively regenerates the catalyst. Preferably, the hydrogen and/or oxygen comprises from 2 to 100 mole percent of the regeneration gas. Suitable inert gases are non-reactive and include, for example, nitrogen, helium, and argon. The time during which the catalyst is regenerated can range from as short as 2 minutes to as long as several hours, for example, about 20 hours at the lower regeneration temperatures. In an alternative embodiment, water is beneficially added to the regeneration gas in an amount preferably ranging from 0.01 to 100 mole percent.

The invention will be further clarified by a consideration of the following examples, which are intended to be purely exemplary of the use of the invention. Other embodiments of the invention will be apparent to those skilled in the art from a consideration of this specification or practice of the invention as disclosed herein. Unless otherwise noted, all percentages are given on a mole percent basis.

### Preparation of Titanium Silicalite TS-1 having Si/Ti = 100

Tetraethylorthosilicate (Fisher TEOS, 832.5 g) was weighed into a 4 liter stainless steel beaker and sparged with nitrogen gas for 30 minutes. Titanium n-butoxide (DuPont, Ti(O-n-Bu)₄) was injected from a syringe into the silicate. The weight of the titanium n-butoxide which was added to the TEOS was 14.07g, taken by difference. A clear yellow solution was formed. The solution was heated and stirred under nitrogen for about 3 h. The temperature varied from 50°C to 130°C. The solution was then chilled in an ice bath.

A 40 wt. percent solution of tetrapropylammonium hydroxide (TPAOH, 710.75 g) was weighed into a polyethylene bottle, which was capped and placed in an ice bath. The TPAOH was added dropwise to the chilled TEOS solution with vigorous stirring by an overhead stirrer. After one-half of the TPAOH had been added, the TEOS solution was cloudy and began to thicken. Within five minutes the solution froze completely. At this point the remainder of the TPAOH was added, the gel was broken up with a spatula, and stirring was resumed. Deionized water (354 g) was added, and the solution was warmed to room temperature. After 5 h the solids had largely dissolved, and an additional quantity of deionized water (708 g) was added. Stirring was continued overnight yielding a clear yellow synthesis gel containing no solids.

The synthesis gel was poured into a 1 gallon (3.785 liters) stainless steel autoclave and sealed. The autoclave was heated to 120°C and then gradually to 160°C where it was kept for 6 days. The reactor contents were stirred at all times. At the end of the reaction period, the autoclave was cooled and a milky white suspension was recovered. The solids were recovered, washed, centrifuged, and re-suspended in deionized water. The solids were filtered, dried at room temperature, heated slowly to 550°C, and calcined thereat for 8 h. The solid was identified as having an MFI structure, as determined by XRD. Raman spectra did not reveal any crystalline titania. A Si/Ti atomic ratio of 100 was found, as measured by X-ray fluorescence (XRF). Yield of titanium silicalite: 106 g.

### Example 1. Preparation of Epoxidation Catalyst

Titanium silicalite TS-1 (10.042 g) having a Si/Ti atomic ratio of 100, prepared as described hereinabove, was added to an aqueous solution of chloroauric acid, HAuCl₄•3H₂O (0.4829 g in 50 ml water). The pH was adjusted to between 7 and 8 by adding sodium carbonate. Magnesium nitrate, Mg(NO₃)₂•6H₂O (1.97 g), was added as was more sodium carbonate until the pH was between 7 and 8. The total amount of sodium carbonate used was 0.62 g. The mixture was stirred overnight. A solid product was filtered, and the filtercake was washed 3 times with 150 ml of water. The wet filtercake was dried at 100°C for 2 h. The dried solid was heated over an 8 h period to 400°C and then calcined under air at 400°C for 5 h to yield an epoxidation catalyst comprising gold on TS-1. Catalyst composition as determined by neutron activation analysis (NAA) was the following: Au, 1.07 percent, Si 41.0 percent, Ti 0.77 percent, Mg 0.21 percent, and Na 0.31 percent (wt percent).
Average gold particle size was 35 Å, as determined by TEM.

### Example 2. Oxidation of Propylene to Propylene Oxide

The epoxidation catalyst of Example 1 was tested in the direct oxidation of propylene to propylene oxide. The catalyst (5 cm³) was loaded into a 10 cm³ fixed-bed, continuous flow reactor with flows of helium, oxygen, hydrogen, and propylene. Total flow rate was 150 cm³/min (or GHSV 1,800 h⁻¹). Feedstream composition was 5.0 percent hydrogen, 10.5 percent oxygen, and 53.6 percent propylene, the balance being helium. Propylene, oxygen and helium were used as pure streams; hydrogen was mixed with helium in a 20 H₂/80 He (v/v) mixture. Pressure was atmospheric; reactor temperature ranged from 50°C to 165°C. Products were analyzed using an on-line gas chromatograph (Chrompack™ Poraplot™ S column, 25 m) with the results shown in Table 1.

**Table 1.**

| **Direct Oxidation of Propylene (PP) to Propylene Oxide (PO) Using Gold on TS-1 (Si/Ti = 100)**^{**a**} | | | | |
|---|---|---|---|---|
| Time (h) | T (°C) | Conv PP (mol %) | Sel PO (mol %) | H₂O/PO |
| 0.5 | 50 | 0.008 | 57.1 | - |
| 1 | 50 | 0.019 | 84.8 | - |
| 3 | 50 | 0.064 | 96.8 | - |
| 7 | 50 | 0.123 | 98.3 | 5.59 |
| 11 | 50 | 0.164 | 99.3 | 2.85 |
| 14 | 50 | 0.160 | 99.0 | 2.88 |
| 19.5 | 60 | 0.211 | 98.8 | 3.40 |
| 22 | 70 | 0.287 | 98.3 | 4.06 |
| 24 | 80 | 0.366 | 97.6 | 4.67 |
| 40 | 80 | 0.200 | 97.5 | 5.26 |
| 60 | 90 | 0.180 | 96.1 | 7.74 |
| 73 | 110 | 0.264 | 91.8 | 12.20 |
| 119^{b} | 110 | 0.128 | 95.0 | 8.32 |
| 140 | 120 | 0.108 | 93.5 | 8.85 |
| 176^{c} | 150 | 0.289 | 88.2 | 20.50 |
| 208 | 165 | 0.423 | 84.6 | 18.00 |
| 258 | 165 | 0.444 | 86.0 | 13.17 |
| 275 | 165 | 0.446 | 85.5 | 15.27 |

| | | | | |
|---|---|---|---|---|
| a. Feedstream (mol %): 5.0% H₂, 10.5% O₂, 53.6% propylene, balance helium; flow 150 cm³/min; pressure atmospheric b. Flow increased at 119 h to 300 cm³/min | | | | |
| c. Flow increased at 176 h to 400 cm³/min. | | | | |

It is seen that a composition comprising gold and magnesium on TS-1 having a Si/Ti molar ratio of 100 is capable of catalyzing the direct oxidation of propylene to propylene oxide. Activity increases with increasing temperature with a propylene conversion of 0.20 percent at 110°C. Selectivity to propylene oxide reaches a maximum of over 99 percent.

### Preparation of Titanium Silicalite TS-1 having Si/Ti = 27

Tetraethylorthosilicate (Fisher TEOS, 1250g) was weighed into a 3 liter Erlenmeyer flask and sparged with nitrogen gas for 30 minutes. Titanium n-butoxide (DuPont, Ti(O-n-Bu)₄, 51.2 g) was injected from a syringe into the TEOS with vigorous stirring. The flask was placed in a 50°C water bath, stirred for 1 h, and left to stand for about 60 h with a nitrogen pad.

A 40 wt percent solution of tetrapropylammonium hydroxide (Sachem TPAOH, 1069.7 g) was added to deionized water (540 g) in a 2 liter beaker and chilled in an ice bath. The TEOS was also chilled in an ice bath. When both solutions were chilled below 10°C, the TEOS solution was transferred to a 4 liter stainless steel beaker equipped with an overhead stirrer. The TPAOH solution was added dropwise by means of an addition funnel. Addition was completed over 5 h to form a clear yellow synthesis gel.

The synthesis gel was poured into a 1 gallon (3.785 liters) stainless steel autoclave and sealed. The autoclave was heated to 100°C for 2 h and then to 140°C for 2 h and then to 160°C for 6 days. The reactor contents were stirred at all times. At the end of the reaction period the autoclave was cooled and a milky white suspension was recovered. The solids were recovered, washed, centrifuged, and resuspended in deionized water. The washing was repeated three times until the pH of the wash water was below pH 9. The solids were dried at 65°C overnight to form white cakes, which were crushed to pass a 20 mesh (0.85 mm) sieve. This solid was heated to 500°C over 8 h and then calcined under air at 500°C for 2 h. The solid was identified by XRD to have an MFI structure. Raman spectra revealed titania in the anatase phase (about 50 percent of the total weight of titanium). The Si/Ti atomic ratio was determined to be 27 by XRF. Yield: 175.5 g.

### Examples 3(a) and 3(b). Preparation of Catalysts and Evaluation in the Oxidation of Propylene to Propylene Oxide.

Two catalysts were prepared as follows. Chloroauric acid was dissolved in water (50 g). TS-1 having a Si/Ti of 27, prepared hereinabove, was added to the solution with stirring. In sample 3(a) sodium carbonate was added. In sample 3(b) sodium carbonate and erbium nitrate were added. The mixtures were stirred for 1 h. Sodium carbonate was added to each mixture to adjust the pH to between 7.0 and 7.6. The solution was stirred for 1.0 h, and the pH was readjusted if needed with sodium carbonate. The mixture was stirred overnight. A solid was filtered from each sample and washed three times with water (150 cm³ per wash). The solid was dried at 110°C for 1 h in air, crushed lightly to break big particles, then calcined in air at 120°C for 3 h. Then, the solid was heated to 400°C over 8 h and held at 400°C for 5 h. Afterwards, the solid was cooled to 350°C for 1 h and then to room temperature to yield a catalyst comprising gold supported on TS-1. The amounts of reagents used are listed for each catalyst.
Ex. 3(a): chloroauric acid, 0.217 g; TS-1, 10.040 g;
   sodium carbonate, 0.218 g;
Ex. 3(b): chloroauric acid, 0.134 g; TS-1, 5.054 g; erbium nitrate,
   1.048 g; sodium carbonate, 0.596 g.
The average size of the gold particles was 30 Å for catalyst 3(a) and 35 Å for catalyst 3(b). Gold loading was about 0.7 wt percent for both catalysts. Erbium loading was 6.5 wt percent, as determined by XRF. TEM showed mixed gold-erbium particles and particles of gold and erbium spread over the surface of the silica.

The catalysts were evaluated in the oxidation of propylene to propylene oxide in a manner similar to that described in Example 2, with the results set forth in Table 2.

**Table 2.**

| **Direct Oxidation of Propylene to Propylene Oxide On Au/TS-1 Catalysts**^{**a**} | | | | | |
|---|---|---|---|---|---|
| Ex | Metal Promoter | T (°C) | Time on Stream (h) | Conv Propylene (mol %) | Sel. PO (mol %) |
| 3(a) | Na | 70 | 15 | 0.09 | 97.5 |
| " | " | 110 | 18 | 0.25 | 95.6 |
| " | " | 140 | 130 | 0.40 | 92.0 |
| 3(b) | Na, Er | 70 | 15 | 0.15 | 98.2 |
| " | " | 110 | 18 | 0.44 | 96.7 |
| " | " | 140^{b} | 130 | 0.45 | 92.0 |

| | | | | | |
|---|---|---|---|---|---|
| a. Feedstream (mol%): 10% H₂, 10% O₂, 30% propylene, balance helium; flow 150 cm³/min, pressure atmospheric; Catalyst 3(a), 10 cm³; Catalyst 3(b), 5 cm³ | | | | | |
| b. Flow increased at 140°C to 200 cm³/min. | | | | | |

Both catalysts are seen to catalyze the direct oxidation of propylene to propylene oxide. When Example 3(a) is compared with Example 3(b) under identical process conditions, it is seen that the catalyst with the erbium promoter has a higher conversion at about the same selectivity. In Example 3(b) conversion reaches 0.44 percent at a selectivity to propylene oxide of 96.7 percent.

### Example 4. Evaluation of Regenerated Catalysts

The used catalysts of Examples 3(a) and 3(b) were removed from the reactors and put into an air oven at 400°C and stirred every 30 min for a total of 2 h to yield regenerated catalysts which were evaluated in the oxidation of propylene to propylene oxide as shown in Table 3, Examples 3(a)-1 and 3(b)-1. The catalysts were regenerated a second time at 220°C in 10 percent oxygen in helium and then cooled to 130°C where they were evaluated in the oxidation process, as shown in Table 3, Examples 3(a)-2 and 3(b)-2. The catalysts were regenerated a third time at 250°C in 10 percent oxygen in helium and then cooled to 130°C where they were evaluated in the oxidation process, as shown in Table 3, Examples 3(a)-3 and 3(b)-3. The erbium promoted catalyst was heated to 385°C overnight in the oxygen/helium mixture and evaluated in the oxidation process as shown in Table 3, Example 3(b)-4.

**Table 3.**

| **Use of Regenerated Catalysts in Direct Oxidation of Propylene to Propylene Oxide**^{**a**} | | | | |
|---|---|---|---|---|
| Ex. | Temp (°C) | Time on Stream (h) | Conv Propylene (mol%) | Sel. PO (mol%) |
| 3(a)-1 | 110 | 2 | 0.18 | 91 |
| " | 110 | 8 | 0.16 | 93 |
| | 110 | 12 | 0.15 | 93 |
| " | 130 | 24 | 0.20 | 92 |
| 3(a)-2 | 130 | 4 | 0.20 | 94 |
| 3(a)-3 | 130 | 4 | 0.24 | 90 |
| 3(b)-1 | 110 | 2 | 0.58 | 97 |
| " | 110 | 8 | 0.30 | 97 |
| " | 110 | 12 | 0.22 | 97 |
| " | 130 | 24 | 0.27 | 96 |
| 3(b)-2 | 130 | 4 | 0.28 | 95 |
| 3(b)-3 | 130 | 4 | 0.26 | 89 |
| 3(b)-4 | 130 | 0.5 | 0.83 | 90 |
| " | 130 | 3.5 | 0.53 | 92 |
| " | 130 | 8 | 0.46 | 93 |

| | | | | |
|---|---|---|---|---|
| a. Feedstream (mol %): 10% H₂, 10% O₂, 30% propylene, balance helium; flow 150 cm³/min, pressure atmospheric. | | | | |

It is seen in Table 3 that catalysts regenerated up to four times continue to exhibit significant activity at high propylene oxide selectivity.

### Example 5. Catalyst Preparation and Epoxidation Process

Three catalysts (A, B, C) comprising gold on TS-1 (Si/Ti = 27) were prepared in a manner similar to Example 1 using the reagent amounts specified in Table 4.

**Table 4.**

| **Quantities of Reagents for Catalyst Preparations** | | | |
|---|---|---|---|
| **Reagent** | **A (g)** | **B (g)** | **C (g)** |
| HAuCl₄.3H₂O | 0.1056 | 0.2089 | 0.4483 |
| TS-1 (Si/Ti = 27) | 10.00 | 10.00 | 10.01 |
| Mg(NO₃)₂.6H₂O | 2.00 | 2.00 | 2.00 |
| Na₂CO₃ | 0.18 | 0.38 | 0.56 |

Catalysts A, B, and C were evaluated in the direct oxidation of propylene as in Example 2 with the results shown in Table 5.

**Table 5.**

| **Direct Oxidation of Propylene With Au/TS-1 Catalyst** | | |
|---|---|---|
| Catalyst | Propylene Conv (mol %) | Sel PO (mol %) |
| A^{a} | 0.09 | 95.2 |
| B^{a} | 0.15 | 92.9 |
| C^{a} | 0.19 | 92.7 |
| C^{b} | 0.87 | 92.0 |

| | | |
|---|---|---|
| a. Catalyst, 5 cm³; Feedstream (mol %): 10% H₂, 10% O₂, 30% propylene, balance helium; 110°C, atmospheric pressure, flow 250 cm³/min | | |
| b. Catalyst (8 cm³) calcined at 375°C for 3 h in air. Feedstream (mol %): 6.5% H₂, 6.5% O₂, 35% propylene, balance helium; 90°C, 185 psia (1276 kPa), flow 943 cm³/min. | | |

It is seen in Table 5 that at constant temperature, pressure and flow rate, as the gold loading is increased, the conversion of propylene also increases. It is also seen that as the pressure of the process is increased, the conversion of propylene is significantly increased. Selectivity remains about constant at greaterthan 90 percent under the process conditions shown.

### Examples 6 (a-j).

Ten catalysts were prepared as follows: Chloroauric acid (1.4526 g) was dissolved in water (500.0 cm³). The total solution was divided into 10 portions of 50 cm³ each. A TS-1 support (Ti/Si = 31) was crushed to greater than 60 mesh (0.25mm). The TS-1, in the amount shown in Table 6, was added to 50.0 cm³ of the gold solution, and the resulting suspension was stirred at room temperature for about 30 min. A promoter metal salt in the amount shown in Table 6 was added to each mixture, and the mixture was stirred for 1 h.

**Table 6.**

| **Catalyst Preparation** | | |
|---|---|---|
| Exp. 6 | TS-1, g | Promoter (g) in addition to Na |
| a | 5.04 | none |
| b | 5.01 | Mg(NO₃)₂.6H₂O (0.5050 g) |
| c | 5.00 | Ca(NO₃)₂.4 H₂O (0.4648 g) |
| d | 5.04 | Ba(NO₃)₂ (0.5128 g) |
| e | 5.03 | La(NO₃)₃.6 H₂O (0.8472 g) |
| f | 5.02 | Pr(NO₃)₃.6 H₂O (0.8859 g) |
| g | 5.05 | Eu(NO₃)₃.6 H₂O (0.9022 g) |
| h | 5.03 | Dy(NO₃)₃.5 H₂O (0.8887 g) |
| i | 5.03 | Er(NO₃)₃.5 H₂O (0.9023 g) |
| j | 5.03 | Lu(NO₃)₃.x H₂O (0.9152 g) |

Sodium carbonate was added until the pH was 7.6 and the mixture was stirred for 1 h. If necessary, more sodium carbonate was added to raise the pH to 7.6. The mixture was stirred overnight and then allowed to sit over the weekend at room temperature. The mixture was filtered. The filtered material was washed with water, then dried at 120°C in air, then calcined in air over 8 h to 400°C, and held at 400°C for 5 h. Each of the catalysts (5 cm³) was tested in the oxidation of propylene with a flow of 150 cm³/min of 30 percent propylene, 10 percent oxygen, 10 percent hydrogen, with the balance helium. Results are set forth in Tables 7 and 8.

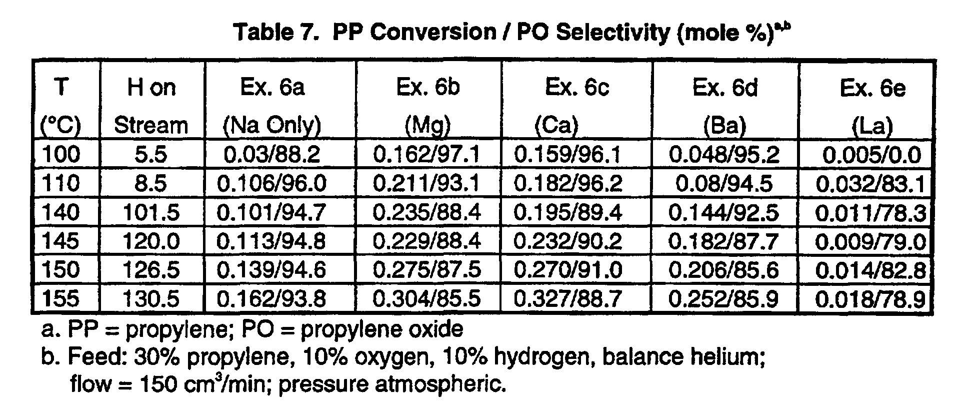

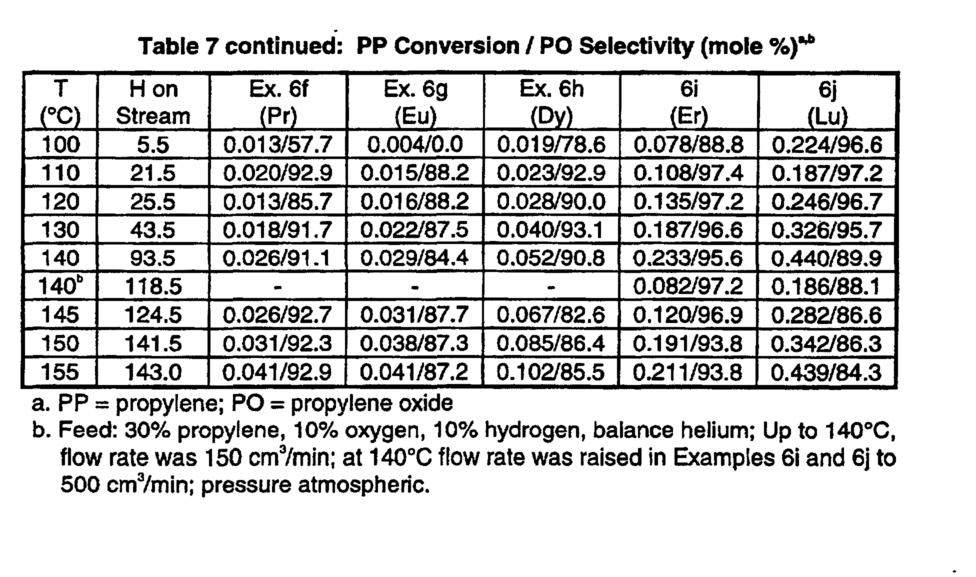

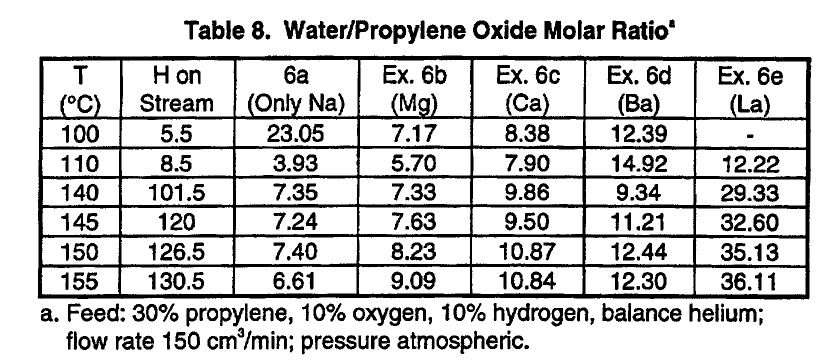

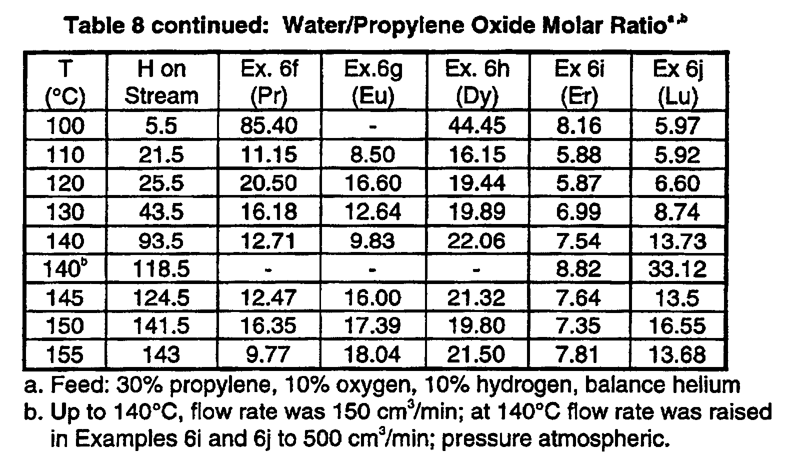

It is seen in Tables 7 and 8 that catalysts containing gold and Group 2 or rare earth lanthanide metals supported on TS-1 are active catalysts for the oxidation of propylene to propylene oxide.

### Examples 7 (a-d).

Five catalysts were prepared as follows: Chloroauric acid (1.4539 g) was dissolved in water (500.0 cm³). A 50 cm³ portion of the gold solution was used to make each catalyst sample. A TS-1 support (Ti/Si = 31; greater than 60 mesh; 0.25 mm) was added in the following amount to the solution: (a) 5.03 g; (b) 5.03 g; (c) 5.03 g; and (d) 5.05 g. The resulting suspension was stirred at room temperature for about 1 h. The pH of the solution was adjusted to 7.6 with one of the following carbonate salts (a) lithium carbonate; (b) potassium carbonate; (c) rubidium carbonate; and (d) cesium carbonate. The mixture was stirred for 1 h and then more carbonate salt was added if necessary to raise the pH to 7.6. The mixture was stirred overnight at room temperature. The mixture was filtered, and the filtered material was washed with water (150 cm³). The wet solid was dried at 120°C in air and then calcined in air over 8 h to 400°C and held at 400°C for 5 h. Each of the catalysts (5 cm³) was tested in the oxidation of propylene with a flow of 150 cm³/min of 30 percent propylene, 10 percent oxygen, 10 percent hydrogen, with the balance helium. Results are set forth in Tables 9 and 10.

**Table 9.**

| **PP Conversion / PO Selectivity (mole %)**^{**a,b,c**} | | | | | | |
|---|---|---|---|---|---|---|
| T (°C) | H on Stream | CE 1 (NH₄) | Ex. 7a (Li) | Ex. 7b (K) | Ex. 7c (Rb) | Ex. 7d (Cs) |
| 100 | 4.5 | .060/73.0 | .014/82.8 | .048/92.1 | .039/90.4 | .039/90.4 |
| 110 | 7.5 | .095/78.4 | .064/95.5 | .120/96.9 | .070/94.6 | .099/95.7 |
| 110 | 24 | .075/67.3 | .042/95.1 | .075/97.1 | .055/95.7 | .070/98.1 |
| 120 | 28 | .099/68.7 | .041/96.7 | .092/96.4 | .072/95.6 | .093/96.9 |
| 120 | 48.5 | .074/72.2 | .026/99.9 | .090/96.5 | .071/95.3 | .091/97.4 |
| 130 | 54 | .122/56.1 | .034/95.8 | .122/96.1 | .101/95.0 | .125/96.2 |
| 130 | 69 | .119/54.0 | .030/95.2 | .131/96.0 | .111/94.9 | .128/96.2 |
| 130 | 71.5 | .061/68.7* | | | | |
| 140 | 73 | .086/65.1* | .039/95.1 | .163/95.2 | .135/94.2 | .179/95.8 |
| 145 | 79 | .131/61.6* | .037/94.9 | .194/94.6 | .163/93.4 | .222/95.3 |
| 145 | 93.5 | .105/56.0* | .035/94.7 | .198/94.6 | .178/93.8 | .226/95.4 |
| 150 | 99.5 | .163/58.8* | .040/93.1 | .225/94.4 | .203/93.1 | .273/94.9 |
| 155 | 105 | | .045/92.8 | .250/94.1 | .230/92.3 | .320/94.2 |
| 160 | 117.5 | | .050/92.3 | .290/93.6 | .270/89.0 | .366/93.7 |
| 165 | 122.5 | | .053/93.1 | .323/92.8 | .297/91.3 | .465/92.9 |
| 170 | 143.5 | | .061/93.0 | .364/90.0 | .331/88.3 | .479/91.8 |
| 175 | 149 | | .074/84.9 | .405/88.9 | .369/86.7 | .539/91.2 |

| | | | | | | |
|---|---|---|---|---|---|---|
| a. PP = propylene; PO = propylene oxide b. Feed: 30% propylene, 10% oxygen, 10% hydrogen, balance helium; flow rate =150 cm³/min; pressure atmospheric c. Asterisk refers to a flow rate increase to 500 cm³/min for the ammonium material due to high water production. | | | | | | |

**Table 10.**

| **H**_{**2**}**O/PO Molar Ratio**^{**a,b**} | | | | | | |
|---|---|---|---|---|---|---|
| T (°C) | H on Stream | CE 1 (NH₄) | Ex. 7a (Li) | Ex. 7b (K) | Ex. 7c (Rb) | Ex. 7d (Cs) |
| 100 | 4.5 | 38.29 | 50.38 | 12.71 | 8.92 | 10.72 |
| 110 | 7.5 | 28.78 | 8.90 | 5.49 | 7.43 | 5.90 |
| 110 | 24 | 40.95 | 8.62 | 3.47 | 7.35 | 4.45 |
| 120 | 28 | 48.57 | 10.28 | 6.05 | 5.80 | 5.42 |
| 120 | 48.5 | 60.97 | 13.21 | 6.97 | 8.11 | 5.98 |
| 130 | 54 | 94.93 | 14.52 | 6.60 | 7.60 | 5.32 |
| 130 | 69 | 98.42 | 15.2 | 7.33 | 6.64 | 5.31 |
| 130 | 71.5 | 40.80* | | | | |
| 140 | 73 | 47.74* | 11.54 | 6.67 | 6.64 | 5.23 |
| 145 | 79 | 56.81* | 13.64 | 6.95 | 6.74 | 5.58 |
| 145 | 93.5 | 64.88* | 12.33 | 7.40 | 7.52 | 6.09 |
| 150 | 99.5 | 64.00* | 16.59 | 8.16 | 6.73 | 5.68 |
| 155 | 105 | | 16.80 | 8.04 | 7.79 | 5.84 |
| 160 | 117.5 | | 13.81 | 7.82 | 8.88 | 6.67 |
| 165 | 122.5 | | 11.97 | 9.51 | 7.79 | 6.46 |
| 170 | 143.5 | | 10.08 | 9.90 | 9.84 | 9.45 |
| 175 | 149 | | 11.87 | 11.43 | 11.24 | 10.56 |

| | | | | | | |
|---|---|---|---|---|---|---|
| a. Feed: 30% propylene, 10% oxygen, 10% hydrogen, balance helium; flow rate = 150 cm³/min; pressure atmospheric b. Asterisk refers to a flow rate increase to 500 cm³/min for the ammonium material due to high water production. | | | | | | |

It is seen in Tables 9 and 10 that a catalyst comprising gold and optionally a Group 1 promoter metal supported on a porous titanosilicate is an active catalyst for oxidizing propylene with oxygen to propylene oxide.

### Comparative Experiment 1 (CE 1)

A catalyst is prepared as in Example 7 with the exception that 5.05 g of TS-1 are used and ammonium carbonate is employed to adjust the pH. No promoter metal is employed. The catalyst is tested in the oxidation of propylene as in Example 7 with the results shown in Tables 9 and 10. When Examples 6(a-j) and Examples 7(a-d) are compared with Comparative Experiment 1, it is concluded that the presence of a promoter metal in the catalyst increases propylene oxide selectivity and significantly decreases the production of water. In some instances, conversion is also increased. The concentration of gold in the comparative experiment was 1.28 weight percent. By comparison, the quantity of gold in the metal promoted catalysts of Examples 6(a-j) and 7(a-d) was less. Accordingly, the use of the promoter metals in these examples appears to lead to more efficient gold usage.

In the following Example 8, the titanium K-edge XANES spectrum was recorded as follows. The Ti K-edge XANES data were collected on beam line X19A at the National Synchrotron Light Source (NSLS). The monochromator was a NSLS boomerang-type flat crystal monochromator with Si(111) crystals. Mirrors were used to focus the beam both horizontally and vertically resulting in an approximately 1 mm x 1 mm beam size at the focal position inside an experimental hutch. A 0.4 mm white beam slit was used to enhance the resolution. The synchrotron operated with an electron energy of 2.583 GeV with beam currents ranging from 100 to 300 mA. Higher harmonics in the beam were rejected by detuning the second Si(111) monochromator crystals to 75 percent of the maximum intensity. The incident beam intensity was monitored with an ion chamber which was integral to the beam pipe and continuously purged with helium gas. The X-ray absorption spectra were recorded as fluorescence yield spectra using a Lytle *in situ* cell flushed with nitrogen gas. No fluorescence filter was used although Soller slits were in place. The sample chamber was placed close to the end of the beam pipe to minimize air absorption and scattering at the relatively low energy of the Ti K-edge (4.996 keV). All of the catalyst samples were measured using the Lytle *in situ* cell arrangement with the catalyst powders pressed into self-supporting 1 inch (2.54 cm) diameter wafers (typical parameters used: 0.3-0.4 g catalyst, 3500 Kg for 5 min.)

The instrument was run in transmission mode. Titanium foil was used to calibrate the energy as follows. The first maximum of the first derivative of the metallic titanium K-edge was set at 4966.0 eV. Measurement of the sample energy was made relative to the 4966.0 eV calibration point, which was taken as zero energy.

As a correlation with the Raman measurement technique, some samples were heated at 500°C in a 20 percent mixture of oxygen in helium prior to XANES analysis. Due to the location of the thermocouple in the Lytle cell, it is believed that the actual catalyst temperature could be as much as 50°C lower than the set point. After the treatment the cell was purged with pure helium to minimize absorption of the X-rays by oxygen.

### Example 8.

A support comprising titanium dispersed on silica is prepared following the procedure of S. Srinivasan et al. as described in the *Journal of Catalysis* **131**, 260-275 (1991), with the exception that the titanium-silica composite is not heated to a temperature greater than 200°C . Cabosil silica is used. Neutron activation analysis (NAA) of the support gives 2.84 percent Ti and 44 percent silicon. The surface area of the support is 300 m²/g. The support shows no crystalline phases of titania as detected by Raman and Ti K-edge XANES. Ti K-edge XANES exhibits one peak at +4.8 eV relative to an internal metallic titanium standard set at 4,966.0 eV. Gold is deposited on the support as follows: Chloroauric acid (0.04 g) is dissolved in water (100 ml). The pH of the solution is adjusted to 7.5 at 80°C with sodium carbonate. Then the support (1.0 g) is added and stirred. The mixture is cooled to room temperature and magnesium nitrate (0.1 g) is added. The mixture is stirred overnight at room temperature. A solid material is filtered and washed once with water. The solid is calcined in air by heating to 400°C over 8 h. and holding thereat for 3 h. Afterwards, the solid is cooled to room temperature.

Composition by NAA (weight percent): 2.86 percent Ti, 45.0 percent Si, 0.25 percent Au, 0.54 percent Mg, and 0.33 percent Na. HR-TEM exhibits no organized structure indicative of crystalline titanium dioxide. Raman spectrum exhibits no peaks for crystalline titanium dioxide. Average gold particle size is 27 Å, as measured by HR-TEM.

The catalyst (1 g) is loaded into a 10 cm³ fixed-bed, continuous flow reactor with feeds of helium, oxygen, hydrogen, and propylene. Feedstream composition is 30 percent propylene, 7 percent hydrogen, 7 percent oxygen, the balance helium. The propylene/hydrogen molar ratio is 4.2; the propylene/oxygen ratio is 4.2; the hydrogen/oxygen ratio is 1.0. Propylene, oxygen and helium are used as pure streams; hydrogen is mixed with helium in a 20 H₂/80 He (v/v) mixture. Total flow rate is 2400 cm³/h. Pressure is atmospheric; reactor temperature 135°C. Products are analyzed using on-line gas chromatography (Chrompak™ Porapak™ S, 25 m) and on-line mass spectrometry.

The catalyst exhibits a 2 percent propylene conversion at 92 percent selectivity to propylene oxide for 20 h at 145°C. The maximum conversion is 3.3 percent at 92 percent selectivity to propylene oxide, the only detectable byproducts being carbon dioxide and water. The catalyst produces greater than 0.58 mmol/g cat-h for 20 h with the peak at 1.0 mmol/g cat-h. The outlet propylene oxide concentration is greater than 0.6 percent for 20 h with the peak at 1 percent

### Example 9.

In a glovebox titanium ethoxide [1.14 g, Ti(O-Et)₄ (∼20 (wt) percent Ti in ethanol) from Aldrich] was dissolved in hexane (20.8 g). The resulting solution was added to silica (11.1 g, 40/60 mesh (0.25-0.42 mm) of Cabot Cab-O-Sil - EH5 fumed silica). The silica had previously been wetted and dried at 110°C, and calcined at 500°C. The mixture was shaken and allowed to sit for 10 min. The solvent and volatiles were removed on a rotary evaporator at room temperature for 1 h in vacuo. Then, the residue was heated to 100°C under vacuum, rotated at 100°C for about 1 h, and cooled to room temperature to obtain a support of this invention.

A gold solution was made by dissolving chloroauric acid (0.1040 g) in water (400 cm³) and heating to 70°C. The pH was adjusted to 7.5 with sodium carbonate. The support (5.017 g) was added quickly and stirred vigorously at 70°C. The pH was readjusted to 7.5 with sodium carbonate. The mixture was stirred at 70°C for 1 h while keeping the pH at 7.5, and then cooled to room temperature. The solids were filtered. The solids were added to water (200 cm³) at pH 7.5 (from Na₂CO₃) and stirred for 5 min. The solids were filtered, dried at room temperature for 1 h by pulling air through the solids on the filter frit. The material was calcined in air from room temperature to 100°C in 1 h; held at 100°C for 1 h; then heated in 8 h to 400°C and held at 400°C for 4 h yielding a catalyst of this invention.

Composition by NAA (weight percent): 0.106 percent Au, 0.48 percent Na, 1.96 percent Ti, 43.2 percent Si; no magnesium detected. No crystalline titanium dioxide was detected by Raman (532 nm excitation) or HR-TEM. Average gold particle size was 15 Å. The UV-VIS DRS (fresh catalyst) exhibited a peak at 309.9 nm. The Ti K-edge XANES exhibited a single peak at +4.70 eV.

The catalyst (2.01 g, 7.5 cm³) was tested in the oxidation of propylene with oxygen with the results shown in Table 3. The used catalyst was regenerated a first time as follows. The catalyst was flushed with a mixture of oxygen (10 percent) in helium until no propylene was seen on a mass spectrometer. The catalyst was then heated from 140°C to 350°C in 45 min in the oxygen/helium mixture at a flow of 150 cm³/min, then held at 350°C for 2 h. The catalyst was cooled to 120°C in the gas mixture. The regenerated catalyst was tested in the oxidation process with the results shown in Table 3. The catalyst was regenerated a second time as follows. The catalyst was flushed with a mixture of oxygen (10 percent) in helium until no propylene was seen on a mass spectrometer. The catalyst was heated from 120°C to 350°C in 1 h in the oxygen/helium mixture at a flow of 150 cm³/min, then heated to 370°C in about 15 min, and held at 370°C for 1 h. The catalyst was cooled to 350°C in the oxygen/helium mixture and held at 350°C for 4 h. The catalyst was cooled to 120°C in the oxygen/helium mixture and then retested in the oxidation process with the results shown in Table 11.

**Table 11.**

| **% PO Sel / % PP Conv / (H**_{**2**}**O/PO)**^{**a,b**} | | | | |
|---|---|---|---|---|
| Time (h) | T (°C) | % PO Sel. | % PP Conv. | H₂O/PO |
| 0.1 | 100 | 96.0 | 0.156 | 7.02 |
| 0.9 | 100 | 96.4 | 0.213 | 3.79 |
| 1.7 | 120 | 94.4 | 0.293 | 3.67 |
| 2.5 | 120 | 94.1 | 0.245 | 4.54 |
| 3.3 | 120 | 94.3 | 0.233 | 4.30 |
| 14.5 | 120 | 94.2 | 0.138 | 6.37 |
| 16.9 | 140 | 91.5 | 0.254 | 6.96 |

| After first regeneration: | | | | |
|---|---|---|---|---|
| 0.1 | 120 | 92.1 | 0.257 | 7.66 |
| 0.9 | 120 | 94.1 | 0.259 | 4.44 |
| 1.7 | 120 | 93.8 | 0.194 | 5.08 |
| 15.3 | 120 | 94.4 | 0.145 | 6.28 |

| After second regeneration: | | | | |
|---|---|---|---|---|
| 0.1 | 120 | 89.8 | 0.185 | 10.00 |
| 0.9 | 120 | 93.5 | 0.254 | 5.58 |
| 1.7 | 120 | 94.0 | 0.215 | 5.60 |
| 2.5 | 120 | 94.0 | 0.215 | 5.44 |
| 4.1 | 120 | 94.5 | 0.184 | 5.25 |
| 13.7 | 120 | 93.3 | 0.112 | 7.39 |
| 15.3 | 140 | 88.8 | 0.206 | 8.08 |

| | | | | |
|---|---|---|---|---|
| a. % PP conv = mole percentage propylene conversion, % PO Sel = mole percentage selectivity to propylene oxide, H₂O/PO = molar ratio of water to propylene oxide b. Feed: 30% PP, 10% oxygen, 11% hydrogen, balance helium, total flow 150 cm³/min, atmospheric pressure. | | | | |

It is seen that the catalyst of Example 8 containing gold and sodium on a support prepared with titanium ethoxide is an active catalyst for the direct oxidation of propylene to propylene oxide.

### Example 10.

A gold solution was made by dissolving chloroauric acid (0.1055 g) in water (400 cm³) and heating to 70°C. The pH was adjusted to 7.5 with sodium carbonate. The support (5.035 g) of Example 9 was added quickly and stirred vigorously at 70°C. The pH was readjusted to 7.5 with sodium carbonate. Magnesium nitrate (0.50 g) was added to the solution and the pH adjusted with sodium carbonate. The mixture was stirred at 70°C for 1 h while keeping the pH at 7.5, and then cooled to room temperature. The solids were filtered. The solids were added to water (200 cm³) at pH 7.5 (from Na₂CO₃) and stirred for 5 min. The solids were filtered, dried at room temperature for 1 h by pulling air through the solids on the filter frit. The material was calcined in air from room temperature to 100°C in 1 h; held at 100°C for 1 h; then heated in 8 h to 400°C and held at 400°C for 4 h yielding a catalyst of this invention.

Composition by NAA (weight percent): 0.207 percent Au, 0.53 percent Mg, 0.17 percent Na, 1.94 percent Ti, 42.0 percent Si. No crystalline titanium dioxide was detected by Raman (532 excitation). The UV-VIS DRS (fresh catalyst) exhibited a peak at 306.4 nm. Ti K-edge XANES exhibited a single peak at +4.67 eV.

The catalyst (2.01 g, 7.5 cm³) was tested in the oxidation of propylene with oxygen with the results shown in Table 12. The catalyst was regenerated twice as described in Example 9 and retested in the oxidation process with the results shown in Table 12.

**Table 12.**

| **% Set PO / % PP Conv / (H**_{**2**}**O/PO)**^{**a,b**} | | | | |
|---|---|---|---|---|
| Time (h) | T (°C) | % Sel PO | % PP Conv. | H₂O/PO |
| 0.3 | 100 | 97.5 | 0.363 | 5.15 |
| 1.1 | 100 | 97.8 | 0.306 | 4.76 |
| 1.9 | 120 | 91.7 | 0.409 | 6.18 |
| 2.7 | 120 | 94.1 | 0.345 | 6.71 |
| 3.5 | 120 | 91.9 | 0.334 | 6.89 |
| 14.7 | 120 | 94.2 | 0.172 | 9.65 |
| 17.1 | 140 | 84.3 | 0.279 | 12.17 |

| After first regeneration: | | | | |
|---|---|---|---|---|
| 0.3 | 120 | 91.5 | 0.461 | 6.91 |
| 1.1 | 120 | 93.9 | 0.354 | 6.88 |
| 1.9 | 120 | 92.9 | 0.301 | 7.62 |
| 15.5 | 120 | 92.9 | 0.153 | 10.18 |

| After second regeneration: | | | | |
|---|---|---|---|---|
| 0.3 | 120 | 91.9 | 0.549 | 6.74 |
| 1.1 | 120 | 91.3 | 0.390 | 7.42 |
| 1.9 | 120 | 93.2 | 0.315 | 8.00 |
| 2.7 | 120 | 92.8 | 0.252 | 8.72 |
| 4.3 | 120 | 93.4 | 0.244 | 8.46 |
| 13.9 | 120 | 93.9 | 0.167 | 11.39 |
| 15.5 | 140 | 82.8 | 0.276 | 13.80 |

| | | | | |
|---|---|---|---|---|
| a. % PP conv = mole percentage propylene conversion, % PO Sel = mole percentage selectivity to propylene oxide, H₂O/PO = molar ratio of water to propylene oxide b. Feed: 30% PP, 10% oxygen, 11% hydrogen, balance helium, total flow rate 150 cm³/min, atmospheric pressure | | | | |

It is seen that the catalyst of Example 10 containing gold, sodium, and magnesium on a support prepared from titanium ethoxide achieved a high selectivity to propylene oxide, good propylene conversion, and high hydrogen efficiency.

### Example 11.

A support was prepared as in Example 9 with the exception that titanium isopropoxide (1.34 g) dissolved in isopropanol (24.0 g) was used in place of titanium ethoxide dissolved in hexane. Gold was deposited on the support as in Example 9 with the exception that chloroauric acid (0.1050 g) was used with the support (5.045 g).

Composition by NAA (weight percent): 0.098 percent Au, 0.43 percent Na, 1.89 percent Ti, 42.0 percent Si; Mg not detected. No crystalline titanium dioxide was detected by Raman (532 excitation) and HR-TEM. Average gold particle size was 15Å. The UV-VIS DRS (fresh catalyst) exhibited a peak at 301.5 nm. Ti K-edge XANES exhibited a peak at +4.42 eV.

The catalyst (2.0 g, 7.5 cm³) was tested in the oxidation of propylene with oxygen with the results shown in Table 13. The used catalyst was regenerated twice as in Example 9 and retested in the oxidation process with the results shown in Table 13.

**Table 13.**

| **% PO Sel I % PP Conv / (H**_{**2**}**O/PO)**^{**a,b**} | | | | |
|---|---|---|---|---|
| Time (h) | T (°C) | % PO Sel. | % PP Conv. | H₂O/PO |
| 0.5 | 100 | 97.3 | 0.292 | 3.68 |
| 1.3 | 100 | 96.5 | 0.226 | 3.55 |
| 2.1 | 120 | 95.3 | 0.327 | 3.98 |
| 2.9 | 120 | 95.5 | 0.298 | 3.84 |
| 3.7 | 120 | 95.2 | 0.281 | 3.95 |
| 14.9 | 120 | 94.9 | 0.182 | 4.82 |
| 17.3 | 140 | 92.6 | 0.309 | 5.49 |

| After first regeneration: | | | | |
|---|---|---|---|---|
| 0.5 | 120 | 92.2 | 0.366 | 3.85 |
| 1.3 | 120 | 94.9 | 0.279 | 4.07 |
| 2.1 | 120 | 94.8 | 0.245 | 4.27 |
| 15.7 | 120 | 94.4 | 0.146 | 6.59 |

| After second regeneration: | | | | |
|---|---|---|---|---|
| 0.5 | 120 | 90.7 | 0.359 | 4.29 |
| 1.3 | 120 | 94.2 | 0.267 | 4.69 |
| 2.1 | 120 | 94.7 | 0.236 | 5.05 |
| 2.9 | 120 | 94.9 | 0.213 | 4.86 |
| 4.5 | 120 | 94.1 | 0.196 | 5.69 |
| 14.1 | 120 | 93.9 | 0.143 | 7.36 |
| 15.7 | 140 | 91.7 | 0.257 | 6.13 |

| | | | | |
|---|---|---|---|---|
| a. % PP conv = mole percentage propylene conversion, % PO Sel = mole percentage selectivity to propylene oxide, H₂O/PO = molar ratio of water to propylene oxide b. Feed: 30% PP, 10% oxygen, 11% hydrogen, balance helium, total flow rate 150 cm³/min, atmospheric pressure. | | | | |

It is seen that the catalyst of Example 11 containing gold and sodium on a support prepared from titanium isopropoxide achieved excellent selectivity to propylene oxide, good propylene conversion, and excellent hydrogen efficiency.

### Example 12.

Gold was deposited onto the support of Example 11 (5.045 g) in the manner described in Example 10. Chloroauric acid (0.1044 g) was used to prepare the gold solution, and magnesium nitrate (0.49 g) was added to the mixture.

Composition by NAA (weight percent): 0.210 percent Au, 0.48 percent Mg, 0.14 percent Na, 1.85 percent Ti, 41.2 percent Si. No crystalline titanium dioxide was detected by Raman (532 excitation). The DRS (fresh catalyst) exhibited a peak at 298.1 nm. Ti K-edge XANES exhibited a peak at +4.66 eV.

The catalyst (2.00 g, 7.5 cm³) was tested in the oxidation of propylene with oxygen with the results shown in Table 14. The used catalyst was regenerated twice in the manner described in Example 9 and retested in the oxidation process with the results shown in Table 14.

**Table 14.**

| **% PO Sel / % PP Conv / (H**_{**2**}**O/PO)**^{**a,b**} | | | | |
|---|---|---|---|---|
| Time (h) | T (°C) | % PO Sel. | % PP Conv. | H₂O/PO |
| 0.7 | 100 | 96.5 | 0.452 | 4.12 |
| 1.5 | 100 | 96.1 | 0.374 | 4.55 |
| 2.3 | 120 | 91.4 | 0.482 | 6.48 |
| 3.1 | 120 | 90.6 | 0.366 | 6.53 |
| 3.9 | 120 | 92.6 | 0.324 | 6.65 |
| 15.1 | 120 | 92.8 | 0.209 | 8.29 |
| 17.5 | 140 | 85.2 | 0.326 | 12.14 |

| After first regeneration: | | | | |
|---|---|---|---|---|
| 0.7 | 120 | 90.3 | 0.510 | 6.44 |
| 1.5 | 120 | 89.8 | 0.398 | 7.00 |
| 2.3 | 120 | 92.2 | 0.344 | 7.78 |
| 15.9 | 120 | 92.5 | 0.192 | 9.80 |

| After second regeneration: | | | | |
|---|---|---|---|---|
| 0.7 | 120 | 90.3 | 0.504 | 7.10 |
| 1.5 | 120 | 92.0 | 0.400 | 7.24 |
| 2.3 | 120 | 89.1 | 0.368 | 7.59 |
| 3.1 | 120 | 88.8 | 0.323 | 7.81 |
| 4.7 | 120 | 92.7 | 0.271 | 8.57 |
| 14.3 | 120 | 91.9 | 0.198 | 9.73 |
| 15.9 | 140 | 77.8 | 0.336 | 12.68 |

| | | | | |
|---|---|---|---|---|
| a. % PP conv = mole percentage propylene conversion, % PO Sel = mole percentage selectivity to propylene oxide, H₂O/PO = molar ratio of water to propylene oxide b. Feed: 30% PP, 10% oxygen, 11% hydrogen, balance helium, total flow rate 150 cm³/min, atmospheric pressure. | | | | |

It is seen that the catalyst of Example 12 containing gold, sodium, and magnesium on a support prepared from titanium isopropoxide achieved an excellent selectivity to propylene oxide, high conversion of propylene, and good hydrogen efficiency.

### Example 13 (A-E).

Five catalysts were prepared as follows using a titanium dioxide (anatase) support and the quantities of reagents listed in Table 15. Chloroauric acid was dissolved in water (100 g). The titanium dioxide (Degussa P25) was added and stirred. The promoters were added and stirred. Sodium carbonate was added until the pH was between 7.0 and 7.6. The mixture was stirred overnight. The solids were filtered and washed three times with water and dried at 120°C overnight. The samples were calcined in air from 120°C to 400°C in 4 h and held at 400°C for 5 h.

**Table 15.**

| **Reagents for Example 13.** | | | | | |
|---|---|---|---|---|---|
| Ex | Au Cmpd (g) | TiO₂ (g) | Promoter (Pr) | Pr Amt (g) | Na₂CO₃ (g) |
| A | 0.2185 | 10.01 | Ca(NO₃)₂•4H₂O | 1.9707 | 0.25 |
| B | 0.2165 | 10.04 | Ba(NO₃)₂ | 2.0680 | 0.25 |
| C | 0.2190 | 10.01 | La(NO₃)₃•H₂O | 2.0297 | 0.40 |
| D | 0.2180 | 10.03 | Er(NO₃)₃•5H₂O | 2.0020 | 0.40 |
| E | 0.2183 | 10.01 | Lu(NO₃)₃•xH₂O | 2.0102 | 0.40 |

The catalysts were tested in the oxidation of propylene with oxygen to propylene oxide with the results shown in Table 16.

**Table 16.**

| **Oxidation of Propylene (PP) with Oxygen to Propylene Oxide (PO)**^{**a**} | | | | | | |
|---|---|---|---|---|---|---|
| Time (h) | Example 13 | Pr | T (°C) | % PO Sel | % PP Conv | H₂O/PO |
| 0.07 | A | Ca | 50 | 87.8 | 0.203 | 13.42 |
| 0.30 | B | Ba | 50 | 95.9 | 0.419 | 6.45 |
| 0.52 | C | La | 50 | 94.6 | 0.370 | 8.07 |
| 0.75 | D | Er | 50 | 96.6 | 0.134 | 20.11 |
| 0.75 | E | Lu | 50 | 99.3 | 0.113 | 23.68 |
| 1.48 | A | Ca | 50 | 86.8 | 0.278 | 10.75 |

| | Regenerate 1 | | | | | |
|---|---|---|---|---|---|---|
| 0.08 | D | Er | 110 | 51.1 | 0.231 | 20.55 |
| 0.32 | E | Lu | 110 | 56.9 | 0.350 | 13.76 |
| 0.55 | A | Ca | 110 | 63.8 | 0.370 | 11.49 |
| 0.78 | B | Ba | 110 | 50.1 | 0.156 | 37.78 |
| 1.02 | C | La | 110 | 47.6 | 0.134 | 52.57 |

| | Regenerate 2 | | | | | |
|---|---|---|---|---|---|---|
| 0.10 | A | Ca | 80 | 89.4 | 1.960 | 1.28 |
| 0.47 | A | Ca | 80 | 91.9 | 0.756 | 3.91 |
| 0.73 | A | Ca | 80 | 91.2 | 0.543 | 5.54 |
| 1.05 | A | Ca | 80 | 89.6 | 0.430 | 7.32 |
| 1.27 | A | Ca | 80 | 87.9 | 0.330 | 9.81 |
| 1.80 | A | Ca | 80 | 84.3 | 0.219 | 16.14 |
| 2.03 | B | Ba | 80 | 80.8 | 0.123 | 29.93 |
| 2.27 | C | La | 80 | 82.1 | 0.110 | 38.60 |
| 2.50 | D | Er | 80 | 75.4 | 0.160 | 12.48 |
| 2.73 | E | Lu | 80 | 76.4 | 0.131 | 13.46 |

| | Regenerate 3 | | | | | |
|---|---|---|---|---|---|---|
| 0.10 | A | Ca | 80 | 89.6 | 1.923 | 1.37 |
| 0.37 | A | Ca | 80 | 92.8 | 0.800 | 3.53 |
| 1.10 | B | Ba | 80 | 85.6 | 0.233 | 14.15 |
| 1.33 | C | La | 80 | 85.3 | 0.209 | 18.43 |
| 1.58 | D | Er | 80 | 79.3 | 0.234 | 9.55 |
| 1.82 | E | Lu | 80 | 78.9 | 0.182 | 11.22 |
| 2.05 | A | Ca | 80 | 80.8 | 0.171 | 19.65 |

| | Regenerate 4 | | | | | |
|---|---|---|---|---|---|---|
| 0.10 | A | Ca | 80 | 91.0 | 1.436 | 1.81 |
| 0.35 | B | Ba | 80 | 87.2 | 0.595 | 5.10 |
| 0.58 | C | La | 80 | 87.2 | 0.510 | 6.74 |
| 0.82 | D | Er | 80 | 89.4 | 0.320 | 9.39 |
| 1.05 | E | Lu | 80 | 83.7 | 0.238 | 13.45 |

| | Regenerate 5 | | | | | |
|---|---|---|---|---|---|---|
| 0.10 | A | Ca | 80 | 90.9 | 1.466 | 1.75 |
| 0.33 | B | Ba | 80 | 89.4 | 0.702 | 4.26 |
| 0.57 | C | La | 80 | 88.2 | 0.579 | 6.06 |
| 0.80 | D | Er | 80 | 88.4 | 0.311 | 9.72 |
| 1.03 | E | Lu | 80 | 84.7 | 0.251 | 12.94 |

| | | | | | | |
|---|---|---|---|---|---|---|
| a. Feed is 40% PP, 10%O₂, 10% H₂, bal. He; flow is 150 cm³/min; 5 cm³ of catalyst; atmospheric pressure. | | | | | | |

The catalysts were regenerated four times as described hereinafter and retested in the oxidation process after each regeneration with the results shown in Table 16.

Regeneration 1: The catalysts were heated to 300°C in a mixture of oxygen (20 percent) in helium. At 300°C a mixture of hydrogen (20 percent) in helium was started over the catalyst to make water for 30 min. The catalysts were cooled to 110°C and retested in the oxidation process.

Regeneration 2: The catalysts were heated at 10°C/h to 300°C in oxygen (10 percent) in helium, kept at 300°C over the weekend, then cooled to 80°C. Feed started at 80°C.

Regeneration 3: The catalysts were heated at 50°C/h to 300°C in oxygen (10 percent) in helium, kept at 300°C overnight, then cooled to 80°C. Feed started at 80°C.

Regeneration 4: The catalysts were heated to 350°C in oxygen (10 percent) in helium and cooled to 80°C. The catalysts were flushed with a helium mixture containing oxygen (10 percent) and water (2 percent) at 80°C. Then the water was stopped and the feed started at 80°C.

Regeneration 5: The catalysts were heated to 350°C in helium containing oxygen (10 percent) and water (2 percent) and then cooled to 80°C. The water was stopped and the feed started at 80°C.

It is seen in Table 16 that a catalyst prepared from gold on a support containing titanium dioxide and a Group 2 or rare earth metal promoter is an active and selective catalyst for the direct oxidation of propylene to propylene oxide. The catalyst can be regenerated multiple times.

### Example 14.

Three catalyst samples (A-C) were prepared as follows. A gold solution was prepared by dissolving chloroauric acid (1.501 g) in water (1 l). Titania (10 g, Degussa P 25) was added to the gold solution (150 ml) with stirring. The promoter metal shown in Table 17 was dissolved in water (50 ml) and added to the gold/titania mixture with stirring. The mixture was titrated to pH 7.5 with sodium carbonate and stirred for 2 h. The solids were filtered and washed three times with water (100 ml). Sample A was dried at 110°C in an oven. Samples B and C were dried at 60°C in air. The samples were calcined in small tube furnaces in the atmosphere listed in Table 17. The calcination profile was from room temperature to 400°C in 8 h and holding at 400°C for 5 h.

**Table 17.**

| **Reagents for Example 14.** | | | |
|---|---|---|---|
| Ex. | Promoter (Pr) | Pr. Amt (g) | Treatment^{a,b} |
| CE2 | none added (NH₄) | 0.0 | O₂ |
| A | Mg(NO₃)₂·6H₂O | 2.0 | O₂ |
| B | Mg(NO₃)₂·6H₂O | 2.0 | H₂ |
| C | Ca(NO₃)₂·4H₂O | 2.0 | H₂ |

| | | | |
|---|---|---|---|
| a. O₂ calcination in 20 mole percent oxygen in nitrogen b. H₂ calcination in 5 mole percent hydrogen in helium. | | | |

The catalysts were tested in the oxidation of propylene with oxygen to propylene oxide with the results shown in Table 18.

**Table 18.**

| **% PO Sel / % PP Conv / (H**_{**2**}**O/PO)**^{**a,b**} | | | |
|---|---|---|---|
| **Ex.** | **% PO Sel.** | **% PP Conv.** | **H**_{**2**}**O/PO** |
| CE2 | 93.7 | 0.782 | 9.05 |
| A | 97.0 | 3.750 | 0.63 |
| B | 99.7 | 2.380 | 0.68 |
| C | 99.9 | 2.050 | - |

| | | | |
|---|---|---|---|
| a. % PO Sel = mole percentage propylene oxide selectivity; % PP Conv = mole percentage propylene conversion; H₂O/PO = water to propylene oxide molar ratio b. Feed: 30% propylene, 10% oxygen, 10% hydrogen, balance helium; 10 cm³ catalyst; 60°C; atmospheric pressure; GC sample taken when mass spectrometer indicates highest PO production. | | | |

It is seen in Table 18 that a catalyst containing gold on a support of titanium dioxide and a Group 2 promoter metal and prepared by calcination under oxygen or hydrogen is an active and selective catalyst for the direct oxidation of propylene to propylene oxide.

### Comparative Experiment 2.

A catalyst was prepared as in Example 14 with the exception that no promoter metal was added and the pH was adjusted with ammonium carbonate. The catalyst was calcined under oxygen as noted in Table 17. The catalyst was tested in the oxidation of propylene with oxygen to propylene oxide with the results shown in Table 18. When Example 14 is compared with Comparative Experiment 2, it is seen that a catalyst of gold on titanium dioxide containing a promoter metal is significantly more active and selective than a catalyst similarly prepared without the promoter metal.

### Example 15.

A used sample (10 cm³) of the catalyst of Example 14B was regenerated at 230°C and then retested in the propylene oxidation process at 80°C and 98 psia with the results shown in Table 19. The catalyst was regenerated a second time by heating in a mixture of oxygen (10 percent), water (1 percent), and balance helium to 230°C, then cooled to 110°C. The oxygen and water were stopped, and the catalyst was flushed with helium until the water was gone. The catalyst was cooled to 80°C in helium. A flow of propylene (28 percent), oxygen (7 percent) and balance helium were started over the catalyst until the flows were stable. Then the feed was changed to propylene (28 percent), oxygen (7 percent), hydrogen (7 percent), balance helium, and the catalyst was retested in the oxidation process with the results shown in Table 19.

**Table 19.**

| **% PO Sel / % PP Conv / (H**_{**2**}**O/PO)** | | | | | |
|---|---|---|---|---|---|
| Time (h) | T (°C) | P (psia) | % PO Sel | % PP Conv | H₂O/PO |
| After 1st Regeneration:^{a} | | | | | |
| 0.15 | 80 | 98 | 95.9 | 1.452 | 2.58 |
| 0.30 | 80 | 98 | 95.7 | 1.036 | 3.48 |
| 0.58 | 80 | 98 | 93.5 | 0.601 | 5.97 |

| After 2nd Regeneration:^{b} | | | | | |
|---|---|---|---|---|---|
| 0.15 | 80 | 204 | 93.1 | 1.506 | 1.06 |
| 0.30 | 80 | 204 | 95.5 | 0.671 | 2.34 |
| 0.45 | 80 | 204 | 94.1 | 0.448 | 3.58 |
| 1.05 | 80 | 204 | 88.2 | 0.216 | 7.01 |

| | | | | | |
|---|---|---|---|---|---|
| a. Feed: 26.5% propylene (PP), 7% oxygen, 7% hydrogen balance helium; total flow 708 cm³/min; 676 kPa | | | | | |
| b. Feed: 28 % propylene (PP), 7% oxygen, 7% hydrogen, balance helium; total flow 1170 cm³/min; 1407 kPa. | | | | | |

It is seen in Table 19 that a regenerated catalyst comprising gold on a support of titanium dioxide and magnesium achieves good activity and high selectivity to propylene oxide.

### Example 16.

A sample (49.67 g) of silica (PQ CS1040-E, 1/16" extrudates), which had been calcined to 300°C in nitrogen, was impregnated in a glovebox with titanium isopropoxide (4.62 g) in isopropanol (44 g). The impregnated material was kept for 1 h at room temperature in the flask in the glovebox. Then the flask was attached to a rotary evaporator; the solvent and volatiles were removed at room temperature in vacuo. The residue was heated in vacuo to 50°C and held 1 h; then to 80°C and held 1 h; and then finally to 100°C and held 2 h in vacuo. The material, a support comprising titanium dispersed on silica, was cooled to room temperature.

A solution of chloroauric acid (0.40 g) in water (1000 ml) was heated to 60°C; the pH was adjusted to 8.0 with sodium carbonate, and the solution was cooled to room temperature. The titanium-containing support (25 g) was added to the gold solution and mixed overnight on the rotary evaporator at atmospheric pressure. The resulting catalyst was washed with water (300 ml) of pH 8, and dried at 110°C. The catalyst was calcined in air for 3 h to 550°C and held at 550°C for 1 h , then cooled to room temperature.

The catalyst (25 cm³, 10.45 g) was tested in the oxidation of propylene with oxygen using a down-flow reactor. The catalyst was tested under pressure and was regenerated 20 times and was still active. After use, three samples of catalyst were examined by NAA; the samples being taken from different portions of the catalyst bed [top refers to the inlet to the reactor], as shown in Table 20.

**Table 20.**

| **Catalyst Composition (Wt Percent)** | | | | |
|---|---|---|---|---|
| **Sample ID** | **% Si** | **% Ti** | **% Au** | **% Na** |
| Top | 43.4 ± 1 | 1.57 ± 0.03 | 0.39 ± 0.01 | 0.22 ± 0.01 |
| Middle | 43.3 ± 1 | 1.51 ± 0.03 | 0.38 ± 0.01 | 0.22 ± 0.01 |
| Bottom | 44.5 ± 1 | 1.52 ± 0.03 | 0.38 ± 0.01 | 0.21 ± 0.01 |

It is seen in Table 20 that the catalyst maintains a stable composition after 20 cycles regardless of location in the catalyst bed.

During the first ten cycles, the catalyst was used at various temperatures, flow rates, and pressures, and regenerated at 350°C to 400°C. Then the catalyst was tested for another 10 cycles at 120°C using the following regeneration and testing scheme. The catalyst was flushed with O₂ (7 percent) in helium until no propylene was seen on the mass spectrometer. Flow was 2000 cm³/min at a pressure of 200 psi (1379 kPa). Water (0.44 percent) was added to the feed using a pump. The catalyst was heated in the oxygen (7 percent)/water(0.44 percent)/helium mixture to 400°C in about 28 min (approx. rate 600°C/h); held at 400°C for 30 min; then cooled as quickly as possible to 120°C in about 30 min. The water was stopped. The feed was changed to propylene (30 percent), oxygen (7 percent), and helium (balance) for 8 min. The total flow was 2000 cm³/min at 200 psi (1379 kPa). Next, the feed was changed to propylene (30 percent), oxygen (7 percent), hydrogen (7 percent), and helium (balance). The GC sample was taken at the "peak" of the PO production (∼5 min after H₂ was started) and at 60 min later, and overnight for some of the regenerations. Throughout all of the testing cycles the catalyst maintains activity and selectivity, as shown for cycles 11 through 20 in Table 21.

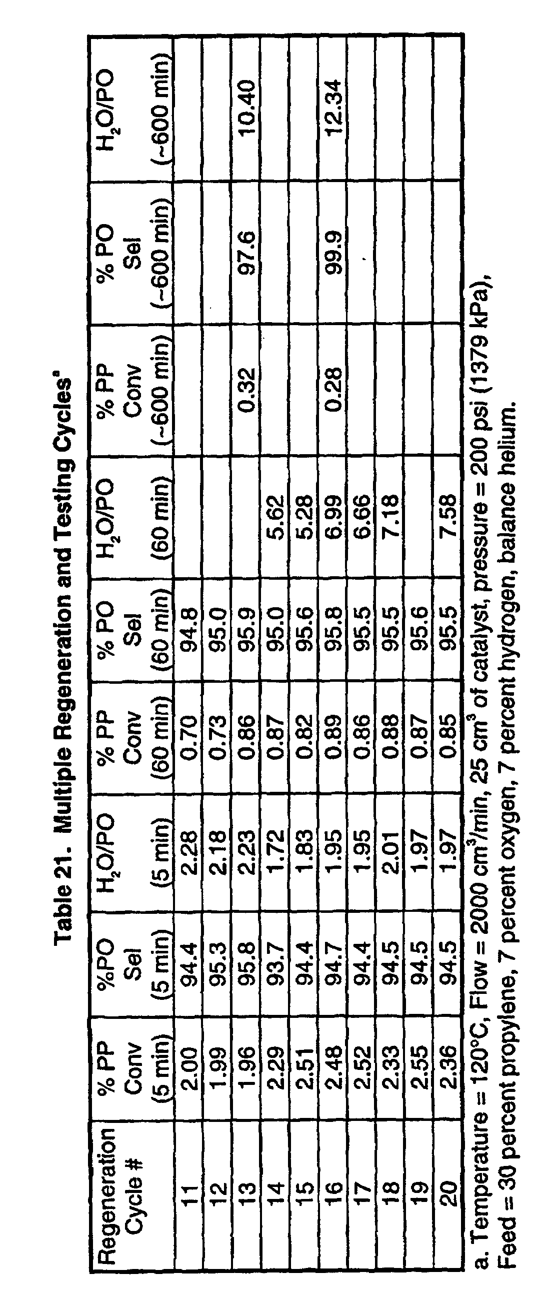

## Claims

1. A process of preparing an olefin oxide comprising contacting an olefin having at least three carbon atoms with oxygen in the presence of hydrogen and an optional diluent, and in the presence of a catalyst comprising gold, at least one promoter metal, and a titanium-containing support.

2. A process of Claim 1, wherein the olefin is a C₃₋₁₂ olefin.

3. A process of Claim 2, wherein the olefin is a C₃₋₈ olefin.

4. A process of Claim 3, wherein the olefin is propylene.

5. A process of any one of the preceding claims, wherein the process is conducted in a vapor phase and a diluent selected from helium, nitrogen, argon, methane, carbon dioxide, steam and mixtures thereof is employed.

6. A process of Claim 5, wherein the diluent is selected from helium, nitrogen, argon, methane, and carbon dioxide.

7. A process of any one of the preceding claims, wherein the process is conducted in a liquid phase and a diluent selected from chlorinated benzenes, C₁₋₁₀ aliphatic alcohols, chlorinated C₁₋₁₀ alkanols, and liquid polyethers, polyalcohols, and polyesters is employed.

8. A process of Claim 7, wherein the diluent is selected from chlorinated benzenes, chlorinated C₁₋₁₀ alkanols, and liquid polyethers, polyalcohols, and polyesters is employed.

9. A process of any one of the preceding claims, wherein the gold has an average particle size of 10 Å or greater.

10. A process of any one of the preceding claims, wherein the gold is loaded onto the support in an amount greater than 0.01 and less than 20 weight percent.

11. A process of Claim 10, wherein the gold is loaded onto the support in an amount of less than 10 weight percent.

12. A process of any one of the preceding claims, wherein the promoter metal is selected from Group 1, Group 2, the lanthanide rare earth metals, the actinide metals, and combinations thereof.

13. A process of Claim 12, wherein the promoter metal is selected from sodium, potassium, rubidium, cesium, magnesium, calcium, barium, erbium, lutetium, and combinations thereof.

14. A process of Claim 12, wherein the promoter metal is selected from magnesium, calcium, barium, erbium, lutetium, lithium, potassium, rubidium, cesium, and combinations thereof.

15. A process of any one of the preceding claims, wherein the promoter metal is loaded onto the support at a loading greater than 0.01 and less than 20 weight percent, based on the weight of the catalyst.

16. A process of any one of the preceding claims, wherein the titanium is in the form of titanium dioxide, titanium dioxide supported on silica, a titanosilicate, a mixture of titanium dioxide and a porous titanosilicate, a promoter metal titanate ,titanium dispersed on silica, titanium dispersed on a promoter metal silicate, or a mixture thereof.

17. A process of Claim 16, wherein the titanium is in the form of titanium dioxide, titanium dioxide supported on silica, a titanosilicate. a promoter metal titanate, titanium dispersed on a promoter metal silicate, or a mixture thereof.

18. A process of any one of the preceding claims, , wherein the process is conducted in a gaseous phase at a temperature greater than 20°C and less than 250°C, a pressure between atmospheric and 2.75 MPa (400 psig), and a gas hourly space velocity of the olefin of greater than 1,000 h⁻¹ and less than 20,000 h⁻¹.

19. A process of Claim 4 for preparing propylene oxide comprising contacting at a temperature greater than 20°C and less than 250°C propylene with oxygen in a gas phase in the presence of hydrogen and an optional diluent and in the presence of a catalyst comprising gold having an average particle size between 10 Å and 200 Å, at least one promoter metal selected from Group 1, Group 2, the lanthanide rare earths, and the actinide metals of the Periodic Table, and a support containing titanium.

20. A catalyst composition comprising gold, at least one promoter metal selected from the Group 1, Group 2, the lanthanide rare earths, and the actinide metals of the Periodic Table of the Elements, and a titanium-containing support, with the proviso that the composition excludes palladium and with the proviso that the composition is exclusive of gold on Group II metal promoter titanates.

21. A composition of Claim 20, wherein the gold is present as particles having an average size of 10 Å or greater.

22. A composition of Claim 20 or Claim 21, wherein the gold is present in an amount greater than 0.01 and less than 20 weight percent.

23. A composition of Claim 22, wherein the gold is loaded onto the support in an amount less than 10 weight percent.

24. A composition of any one of Claims 20 to 23, wherein the promoter metal is sodium, potassium, rubidium, cesium, magnesium, calcium, barium, erbium, lutetium, or combinations thereof.

25. A composition of any one of Claims 20 to 23, wherein the promoter metal is selected from magnesium, calcium, barium, erbium, lutetium, lithium, potassium, rubidium, cesium, and combinations thereof.

26. A composition of any one of Claims 20 to 25, wherein the titanium is in the form of titanium dioxide, titanium dioxide supported on silica, a titanosilicate, titanium dispersed on silica or a mixture thereof.

27. A composition of Claim 26, wherein the titanium is in the form of titanium ions dispersed over the surface of silica with greater than 80 weight percent of the titanium in a disorganized phase.

28. A composition of any one of Claims 20 to 27, wherein the promoter metal is present at a loading greater than 0.01 and less than 20 weight percent, based on the weight of the catalyst composition.

29. A composition of Claim 28, wherein the promoter metal is present in an amount of greater than 0.10 weight percent, based on the weight of the catalyst composition

30. A composition of any one of Claims 20 to 29, wherein the promoter metal(s) comprise sodium.

31. A process for making a composition of any one of Claims 20 to 30 comprising (a) contacting a titanium-containing support with a solution of a gold compound and a solution of at least one promoter metal salt wherein the promoter metal is selected from the Group 1, Group 2, lanthanide rare earths and actinide metals of the Periodic Table, the contacting occurring at a temperature between 20°C and 80°C, then (b) adjusting the pH of the resulting mixture to between 5 and 11 so as to obtain a gold-promoter metal-support composite, (c) optionally washing the composite with no more than 100 ml wash liquid per gram composite, and (d) calcining the mixture under air or under a reducing atmosphere or heating in an inert atmosphere at a temperature between 250°C and 800°C.

32. A process of regenerating a composition of any one of Claims 20 to 30 comprising heating the deactivated catalyst at a temperature between 150°C and 500°C in the presence of a regeneration gas containing hydrogen and/or oxygen and optionally an inert gas.

33. A process of Claim 32, wherein the regeneration gas contains an inert gas.

34. A process of Claim 33, wherein water is added to the regeneration gas.

## Patentansprüche

1. Verfahren zur Herstellung eines Olefinoxids, umfassend das Inkontaktbringen eines Olefins mit mindestens drei Kohlenstoffatomen mit Sauerstoff in der Gegenwart von Wasserstoff und einem optionalen Verdünnungsmittel und in der Gegenwart eines Katalysators, umfassend Gold, mindestens ein Promotormetall und einen Titan enthaltenden Träger.

2. Verfahren nach Anspruch 1, worin das Olefin ein C₃₋₁₂-Olefin ist.

3. Verfahren nach Anspruch 2, worin das Olefin ein C₃₋₈-Olefin ist.

4. Verfahren nach Anspruch 3, worin das Olefin Propylen ist.

5. Verfahren nach einem der vorhergehenden Ansprüche, worin das Verfahren in einer Dampfphase durchgeführt wird und ein Verdünnungsmittel, ausgewählt aus Helium, Stickstoff, Argon, Methan, Kohlendioxid, Dampf und Gemischen davon verwendet wird.

6. Verfahren nach Anspruch 5, worin das Verdünnungsmittel aus Helium, Stickstoff, Argon, Methan und Kohlendioxid ausgewählt wird.

7. Verfahren nach einem der vorhergehenden Ansprüche, worin das Verfahren in einer flüssigen Phase durchgeführt wird und ein Verdünnungsmittel, ausgewählt aus chlorierten Benzolen, aliphatischen C₁₋₁₀-Alkoholen, chlorierten C₁₋₁₀-Alkanolen und flüssigen Polyethem, Polyalkoholen und Polyestem verwendet wird.

8. Verfahren nach Anspruch 7, worin das verwendete Verdünnungsmittel ausgewählt wird aus chlorierten Benzolen, chlorierten C₁₋₁₀-Alkanolen und flüssigen Polyethem, Polyalkoholen und Polyestem.

9. Verfahren nach einem der vorhergehenden Ansprüche, worin das Gold eine. mittlere Teilchengröße von 10 Å oder mehr aufweist.

10. Verfahren nach einem der vorhergehenden Ansprüche, worin das Gold auf den Träger in einer Menge von mehr als 0,01 und weniger als 20 Gew.-% aufgebracht wird.

11. Verfahren nach Anspruch 10, worin das Gold auf den Träger in einer Menge von weniger als 10 Gew.-% aufgebracht wird.

12. Verfahren nach einem der vorhergehenden Ansprüche, worin das Promotormetall ausgewählt wird aus der Gruppe 1, Gruppe 2, den Lanthanidenseltenerdmetallen, den Actinidenmetallen und Kombinationen davon.

13. Verfahren nach Anspruch 12, worin das Promotormetall ausgewählt wird aus Natrium, Kalium, Rubidium, Cäsium, Magnesium, Calcium, Barium, Erbium, Lutetium und Kombinationen davon.

14. Verfahren nach Anspruch 12, worin das Promotormetall ausgewählt wird aus Magnesium, Calcium, Barium, Erbium, Lutetium, Lithium, Kalium, Rubidium, Cäsium und Kombinationen davon.

15. Verfahren nach einem der vorhergehenden Ansprüche, worin das Promotormetall auf den Träger mit einer Beladung von größer als 0,01 und weniger als 20 Gew.-% basierend auf dem Gewicht des Katalysators aufgebracht wird.

16. Verfahren nach einem der vorhergehenden Ansprüche, worin das Titan in der Form von Titandioxid, Titandioxid auf Siliciumdioxidträger, eines Titansilikats, eines Gemischs von Titandioxid und einem porösen Titansilikat, eines Promotormetalltitanats, Titan das auf Siliciumdioxid dispergiert ist, Titan das auf einem Promotormetallsilikat dispergiert ist oder eines Gemisches davon ist.

17. Verfahren nach Anspruch 16, worin das Titan in der Form von Titandioxid, Titandioxid auf Siliciumdioxidträger, eines Titanosilikats, eines Promotormetalltitanats, Titan dispergiert auf einem Promotormetallsilikat oder eines Gemisches davon ist.

18. Verfahren nach einem der vorhergehenden Ansprüche, worin das Verfahren in einer gasförmigen Phase bei einer Temperatur von mehr als 20 °C und weniger als 250 °C, einem Druck zwischen Atmosphärendruck und 2,75 MPa (400 psig) und einer Gasraumgeschwindigkeit pro Stunde des Olefins von größer als 1000 h⁻¹ und weniger als 20.000 h⁻¹ durchgeführt wird.

19. Verfahren nach Anspruch 4 zur Herstellung von Propylenoxid, umfassend das Inkontaktbringen bei einer Temperatur von mehr als 20 °C und weniger als 250 °C von Propylen mit Sauerstoff in einer Gasphase in der Gegenwart von Wasserstoff und einem optionalen Verdünnungsmittel und in der Gegenwart eines Katalysators, umfassend Gold mit einer mittleren Teilchengröße zwischen 10 Å und 200 Å, mindestens eines Promotormetalls, ausgewählt aus der Gruppe 1, Gruppe 2, den Lanthanidenseltenerd- und den Actinidenmetallen des Periodensystems und eines Titan enthaltenden Trägers.

20. Katalysatorzusammensetzung, umfassend Gold, mindestens ein Promotormetall, ausgewählt aus der Gruppe 1, Gruppe 2, den Lanthanidenseltenerd- und den Actinidenmetallen des Periodensystems der Elemente und einen Titan enthaltenden Träger, mit der Maßgabe, dass die Zusammensetzung kein Palladium aufweist und mit der Maßgabe, dass die Zusammensetzung kein Gold auf Gruppe 2-Metallpromotortitanaten aufweist.

21. Zusammensetzung nach Anspruch 20, worin das Gold als Teilchen mit einer mittleren Größe von 10 Å oder mehr vorliegt.

22. Zusammensetzung nach Anspruch 20 oder Anspruch 21, worin das Gold in einer Menge von mehr als 0,01 und weniger als 20 Gew.-% vorliegt.

23. Zusammensetzung nach Anspruch 22, worin das Gold auf den Träger in einer Menge von weniger als 10 Gew.-% aufgebracht ist.

24. Zusammensetzung nach einem der Ansprüche 20 bis 23, worin das Promotormetall Natrium, Kalium, Rubidium, Cäsium, Magnesium, Calcium, Barium, Erbium, Lutetium oder Kombinationen davon ist.

25. Zusammensetzung nach einem der Ansprüche 20 bis 23, worin das Promotormetall ausgewählt ist aus Magnesium, Calcium, Barium, Erbium, Lutetium, Lithium, Kalium, Rubidium, Cäsium und Kombinationen davon.

26. Zusammensetzung nach einem der Ansprüche 20 bis 25, worin das Titan in der Form von Titandioxid, Titandioxid auf Siliciumdioxidträger, eines Titanosilikats, Titan dispergiert auf Siliciumdioxid oder eines Gemisches davon ist.

27. Zusammensetzung nach Anspruch 26, worin das Titan in der Form von Titanionen ist, die über die Oberfläche von Siliciumdioxid verteilt sind, wobei mehr als 80 Gew.-% des Titans in einer nicht organisierten Phase sind.

28. Zusammensetzung nach einem der Ansprüche 20 bis 27, worin das Promotormetall mit einer Beladung von mehr als 0,01 und weniger als 20 Gew.-% basierend auf dem Gewicht der Katalysatorzusammensetzung vorliegt.

29. Zusammensetzung nach Anspruch 28, worin das Promotormetall in einer Menge von mehr als 0,10 Gew.-% basierend auf dem Gewicht der Katalysatorzusammensetzung vorliegt.

30. Zusammensetzung nach einem der Ansprüche 20 bis 29, worin das (die) Promotormetall(e) Natrium umfasst.

31. Verfahren zum Herstellen einer Zusammensetzung nach einem der Ansprüche 20 bis 30, umfassend (a) Inkontaktbringen eines Titan enthaltenden Trägers mit einer Lösung einer Goldverbindung und einer Lösung mindestens eines Promotormetallsalzes, worin das Promotormetall ausgewählt wird aus der Gruppe 1, Gruppe 2, Lanthanidenseltenerd- und Actinidenmetallen des Periodensystems, wobei das Inkontaktbringen bei einer Temperatur zwischen 20 °C und 80 °C erfolgt, dann (b) Einstellen des pH-Werts des resultierenden Gemischs auf zwischen 5 und 11, um einen Goldpromotormetallträgerverbundstoff zu erhalten, (c) gegebenfalls Waschen des Verbundstoffs mit nicht mehr als 100 ml Waschflüssigkeit pro Gramm Verbundstoff und (d) Calcinieren des Gemischs unter Luft oder unter einer reduzierenden Atmosphäre oder Erhitzen in einer inerten Atmosphäre bei einer Temperatur zwischen 250 °C und 800 °C.

32. Verfahren zum Regenerieren einer Zusammensetzung nach einem der Ansprüche 20 bis 30, umfassend das Erhitzen des deaktivierten Katalysators bei einer Temperatur zwischen 150 °C und 500 °C in der Gegenwart eines Regenerationsgases, das Wasserstoff und/oder Sauerstoff und gegebenenfalls ein Inertgas enthält.

33. Verfahren nach Anspruch 32, worin das Regenerationsgas ein inertes Gas enthält.

34. Verfahren nach Anspruch 33, worin Wasser zu dem Regenerationsgas gegeben wird.

## Revendications

1. Procédé de préparation d'un oxyde d'oléfine, comprenant la mise en contact d'une oléfine ayant au moins trois atomes de carbone avec de l'oxygène en présence d'hydrogène et d'un diluant facultatif et en présence d'un catalyseur comprenant de l'or, au moins un métal promoteur et un support contenant du titane.

2. Procédé selon la revendication 1, dans lequel l'oléfine est une oléfine en C₃ à C₁₂.

3. Procédé selon la revendication 2, dans lequel l'oléfine est une oléfine en C₃ à C₈.

4. Procédé selon la revendication 3, dans lequel l'oléfine est le propylène.

5. Procédé selon l'une quelconque des revendications précédentes, dans lequel le procédé s'effectue dans une phase vapeur et un diluant choisi parmi l'hélium, l'azote, l'argon, le méthane, le dioxyde de carbone, la vapeur, et les mélanges de ceux-ci, est employé.

6. Procédé selon la revendication 5, dans lequel le diluant est choisi parmi l'hélium, l'azote, l'argon, le méthane et le dioxyde de carbone.

7. Procédé selon l'une quelconque des revendications précédentes, dans lequel le procédé s'effectue dans une phase liquide et un diluant choisi parmi les benzènes chlorés, les alcools aliphatiques en C₁ à C₁₀, les alcanols en C₁ à C₁₀ chlorés, et les polyéthers, polyalcools et polyesters liquides, est employé.

8. Procédé selon la revendication 7, dans lequel le diluant employé est choisi parmi les benzènes chlorés, les alcanols en C₁ à C₁₀ chlorés, et les polyéthers, polyalcools et polyesters liquides.

9. Procédé selon l'une quelconque des revendications précédentes, dans lequel l'or a une taille de particule moyenne de 10 Å ou supérieure.

10. Procédé selon l'une quelconque des revendications précédentes, dans lequel l'or est chargé sur le support en une quantité supérieure à 0,01% et inférieure à 20% en poids.

11. Procédé selon la revendication 10, dans lequel l'or est chargé sur le support en une quantité inférieure à 10% en poids.

12. Procédé selon l'une quelconque des revendications précédentes, dans lequel le métal promoteur est choisi parmi les métaux du groupe 1, du groupe 2, les métaux de terres rares des lanthanides, les actinides, et les combinaisons de ceux-ci.

13. Procédé selon la revendication 12, dans lequel le métal promoteur est choisi parmi le sodium, le potassium, le rubidium, le césium, le magnésium, le calcium, le baryum, l'erbium, le lutécium, et les combinaisons de ceux-ci.

14. Procédé selon la revendication 12, dans lequel le métal promoteur est choisi parmi le magnésium, le calcium, le baryum, l'erbium, le lutécium, le lithium, le potassium, le rubidium, le césium, et les combinaisons de ceux-ci.

15. Procédé selon l'une quelconque des revendications précédentes, dans lequel le métal promoteur est chargé sur le support à une charge supérieure à 0,01% et inférieure à 20% en poids, ramené au poids du catalyseur.

16. Procédé selon l'une quelconque des revendications précédentes, dans lequel le titane est présent sous la forme de dioxyde de titane, de dioxyde de titane supporté sur de la silice, d'un titanosilicate, d'un mélange de dioxyde de titane et d'un titanosilicate poreux, d'un titanate de métal promoteur, de titane dispersé sur de la silice, de titane dispersé sur un silicate de métal promoteur ou d'un mélange de ceux-ci.

17. Procédé selon la revendication 16, dans lequel le titane est présent sous forme de dioxyde de titane, de dioxyde de titane supporté sur de la silice, d'un titanosilicate, d'un titanate de métal promoteur, de titane dispersé sur un silicate de métal promoteur ou d'un mélange de ceux-ci.

18. Procédé selon l'une quelconque des revendications précédentes, dans lequel le procédé s'effectue dans une phase gazeuse à une température supérieure à 20°C et inférieure à 250°C, à une pression entre la pression atmosphérique et 2,75 MPa (400 psig) et à une vitesse spatiale en gaz de l'oléfine supérieure à 1000 h⁻¹ et inférieure à 20 000 h⁻¹.

19. Procédé selon la revendication 4 pour préparer de l'oxyde de propylène, comprenant la mise en contact, à une température supérieure à 20°C et inférieure à 250°C, de propylène avec de l'oxygène dans une phase gazeuse en présence d'hydrogène et d'un diluant facultatif et en présence d'un catalyseur comprenant de l'or ayant une taille de particule moyenne entre 10 Å et 200 Å, au moins un métal promoteur choisi parmi les métaux du groupe 1, du groupe 2, les terres rares des lanthanides et les actinides de la Classification Périodique des Eléments, et un support contenant du titane.

20. Composition de catalyseur comprenant de l'or, au moins un métal promoteur choisi parmi les métaux du groupe 1, du groupe 2, les terres rares des lanthanides et les actinides de la Classification Périodique des Eléments, et un support contenant du titane, à la condition que la composition exclue le palladium et à la condition que la composition ne comprenne pas d'or sur des titanates de métaux promoteurs du groupe II.

21. Composition selon la revendication 20, dans laquelle l'or est présent sous la forme de particules ayant une taille moyenne de 10 Å ou supérieure.

22. Composition selon la revendication 20 ou la revendication 21, dans laquelle l'or est présent en une quantité supérieure à 0,01% et inférieure à 20% en poids.

23. Composition selon la revendication 22, dans laquelle l'or est chargé sur le support en une quantité inférieure à 10% en poids.

24. Composition selon l'une quelconque des revendications 20 à 23, dans laquelle le métal promoteur est le sodium, le potassium, le rubidium, le césium, le magnésium, le calcium, le baryum, l'erbium, le lutécium ou des combinaisons de ceux-ci.

25. Composition selon l'une quelconque des revendications 20 à 23, dans laquelle le métal promoteur est choisi parmi le magnésium, le calcium, le baryum, l'erbium, le lutécium, le lithium, le potassium, le rubidium, le césium, et les combinaisons de ceux-ci.

26. Composition selon l'une quelconque des revendications 20 à 25, dans laquelle le titane est présent sous la forme de dioxyde de titane, de dioxyde de titane supporté sur de la silice, d'un titanosilicate, de titane dispersé sur de la silice ou d'un mélange de ceux-ci.

27. Composition selon la revendication 26, dans laquelle le titane est présent sous la forme d'ions de titane dispersés sur la surface de la silice, plus de 80% en poids du titane se trouvant dans une phase désorganisée.

28. Composition selon l'une quelconque des revendications 20 à 27, dans laquelle le métal promoteur est présent à une charge supérieure à 0,01% et inférieure à 20% en poids, ramené au poids de la composition de catalyseur.

29. Composition selon la revendication 28, dans laquelle le métal promoteur est présent en une quantité supérieure à 0,10% en poids, ramenée au poids de la composition de catalyseur.

30. Composition selon l'une quelconque des revendications 20 à 29, dans laquelle le métal ou les métaux promoteurs comprennent le sodium.

31. Procédé pour préparer une composition selon l'une quelconque des revendications 20 à 30, comprenant (a) la mise en contact d'un support contenant du titane avec une solution d'un composé d'or et une solution d'au moins un sel de métal promoteur, le métal promoteur étant choisi parmi les métaux du groupe 1, du groupe 2, les terres rares des lanthanides et les actinides de la Classification Périodique des Eléments et la mise en contact se faisant à une température allant de 20°C à 80°C, ensuite (b) l'ajustement du pH du mélange résultant à une valeur entre 5 et 11 de façon à obtenir un composite or/métal promoteur/support, (c) facultativement le lavage du composite avec pas plus de 100 ml de liquide de lavage par gramme de composite, et (d) la calcination du mélange à l'air ou sous une atmosphère réductrice ou le chauffage sous une atmosphère inerte à une température de 250°C à 800°C.

32. Procédé pour régénérer une composition selon l'une quelconque des revendications 20 à 30, comprenant le chauffage du catalyseur désactivé à une température comprise entre 150°C et 500°C en présence d'un gaz de régénération contenant de l'hydrogène et/ou de l'oxygène et optionnellement un gaz inerte.

33. Procédé selon la revendication 32, dans lequel le gaz de régénération contient un gaz inerte.

34. Procédé selon la revendication 33, dans lequel de l'eau est ajoutée au gaz de régénération.
